(19) Europäisches Patentamt
European Patent Office
Office européen des brevets

(11) Veröffentlichungsnummer: **0 338 508 A2**

(12) # EUROPÄISCHE PATENTANMELDUNG

(21) Anmeldenummer: **89106930.4**

(22) Anmeldetag: **18.04.89**

(51) Int. Cl.⁴: **C07K 5/06 , A61K 37/02**

Patentansprüche für folgende Vertragsstaaten: ES + GR

(30) Priorität: **22.04.88 DE 3813819**

(43) Veröffentlichungstag der Anmeldung: **25.10.89 Patentblatt 89/43**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Hock, Franz, Dr.**
**Altstadt 19**
**D-6110 Dieburg(DE)**
Erfinder: **Scholtholt, Josef, Prof. Dr.**
**Höhenstrasse 14**
**D-6450 Hanau 6(DE)**
Erfinder: **Urbach, Hansjörg, Dr.**
**Le Lavandoustrasse 41**
**D-6242 Kronberg/Taunus(DE)**
Erfinder: **Henning, Rainer, Dr.**
**Rotenhofstrasse 31**
**D-6234 Hattersheim am Main(DE)**
Erfinder: **Lerch, Ulrich, Dr.**
**Vorderwart 24**
**D-6238 Hofheim am Taunus(DE)**
Erfinder: **Nickel, Wolf-Ulrich, Dr.**
**Robert-Scholz-Strasse 120**
**D-6232 Bad Soden am Taunus(DE)**
Erfinder: **Rüger, Wolfgang, Dr.**
**Parkstrasse 10**
**D-6233 Kelkheim (Taunus)(DE)**

(54) **Aminosäureester, Verfahren zu ihrer Herstellung, sie enthaltende Arzneimittel und deren Verwendung.**

(57) Die Erfindung betrifft Aminosäureester der Formel I

$$R^3OOC - \underset{R^4}{\overset{*}{CH}} - \underset{R^5}{N} - \underset{O}{\overset{\|}{C}} - \underset{R^1}{\overset{*}{CH}} - NH - \underset{COOR^2}{\overset{*}{CH}} - (CH_2)_n - R \qquad (I)$$

in der n = 2 ist und R, $R^1$, $R^2$, $R^3$, $R^4$ und $R^5$ die in der Beschreibung angegebene Bedeutung haben, ein Verfahren und Zwischenprodukte zu deren Herstellung, diese enthaltende Mittel und deren Verwendung.

EP 0 338 508 A2

# Aminosäureester, Verfahren zu ihrer Herstellung, sie enthaltende Arzneimittel und deren Verwendung

Aus der EP-A-243645 ist die Verwendung von ACE-Hemmern bei der Behandlung cognitiver Dysfunktionen bekannt.

Die Erfindung betrifft neue Aminosäureester der Formel I

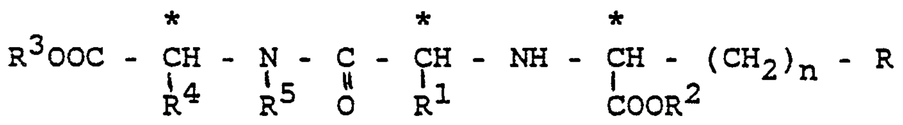

(I)

in welcher

n = 2 ist

R einen gegebenenfalls substituierten aliphatischen Rest mit 1-18 C-Atomen, einen gegebenenfalls substituierten alicyclischen Rest mit 3-20 C-Atomen oder einen gegebenenfalls substituierten aromatischen Rest mit 6-12 C-Atomen bedeutet,

R' die erforderlichenfalls geschützte Seitenkette einer natürlich vorkommenden $\alpha$-Aminosäure der Formel R'-$CH(NH_2)$-COOH bedeutet

$R^2$ Wasserstoff, einen gegebenenfalls substituierten aliphatischen Rest mit 1-18 C-Atomen, Benzyl oder einen gegebenenfalls substituierten alicyclischen Rest mit 3-20 C-Atomen bedeutet,

$R^3$ Wasserstoff, einen gegebenenfalls substituierten aliphatischen Rest mit 1-18 C-Atomen oder einen gegebenenfalls substituierten araliphatischen Rest mit 7-20 C-Atomen bedeutet und

$R^4$ und $R^5$ zusammen mit den sie tragenden Atomen ein mono-, bi- oder tricyclisches heterocyclisches Ringsystem mit 3 bis 15 C-Atomen bilden,

vorausgesetzt daß

A $R^2$ ein n gegebenenfalls substituierten aliphatischen Rest mit 7-18 C-Atomen oder einen gegebenenfalls substituierten alicyclischen Rest mit 7-20 C-Atomen bedeutet und ansonsten n, R, R', $R^3$, $R^4$ und $R^5$ die oben angegebene Bedeutung besitzen,

oder

B $R^3$ einen gegebenenfalls substituierten aliphatischen Rest mit 7-18 C-Atomen oder einen gegebenenfalls substituierten araliphatischen Rest mit 7-20 C-Atomen bedeutet und ansonsten n, R, R', $R^2$, $R^4$ und $R^5$ die oben angegebene Bedeutung besitzen,

oder

C R einen gegebenenfalls substituierten aliphatischen Rest mit 9-18 C-Atomen bedeutet und ansonsten n, R', $R^2$, $R^3$, $R^4$ und $R^5$ die oben genannte Bedeutung besitzen,

und deren physiologisch verträgliche Salze, ausgenommen 2-[N-(1S-Ethoxycarbonyl-3-phenylpropyl)-S-alanyl]-(1S,3S, 5S)-2-azabicyclo[3.3.0]octan-3-carbonsäure-n-octylester, 2-[N-(1S-Carboxy-3-phenylpropyl)-S-alanyl]-(1S,3S,5S)-2-azabicyclo[3.3.0]octan-3-carbonsäure-n-octylester und deren physiologisch verträgliche Salze.

Unter einem gegebenenfalls substituierten aliphatischen Rest versteht man einen aliphatischen acyclischen Rest, d.h. einen Rest mit einer offenen, geraden oder verzweigten Kohlenstoffkette, wie beispielsweise Alkyl, Alkenyl, Alkinyl und entsprechende mehrfach ungesättigte Reste.

Ein gegebenenfalls substituierter alicyclischer Rest ist ein vorzugsweise mono- bis pentacyclischer isocyclischer, nicht aromatischer Rest mit Einfach- oder unsymmetrisch verteilten Doppelbindungen, der auch verzweigt sein (d.h. offenkettige aliphatische Seitenketten tragen) kann, und der über ein Ring-C-Atom oder ein Seitenketten-C-Atom verknüpft ist. Mehrere Ringe als Komponenten eines solchen Restes sind kondensiert, spiroverknüpft oder isoliert. Beispiele für solche Rest sind Cycloalkyl, Cycloalkenyl, Bicycloalkyl, Tricycloalkyl und von mono-, bi- oder oligocyclischen Terpenen abgeleitete Reste, wie Menthyl, Isomenthyl, Bornyl, Caranyl, Epibornyl, Epiisobornyl, Isobornyl, Norbornyl, Neomenthyl, Neoisomenthyl, Pinanyl, Thujanyl; sie sind vorzugsweise unsubstituiert (aliphatische Seitenketten sind nach vorliegender Definition keine Substituenten).

Ein gegebenenfalls substituierter aromatischer Rest ist vorzugsweise Aryl wie Phenyl, Biphenylyl oder Naphthyl, das gegebenenfalls mono-, di- oder trisubstituiert ist. Von Aryl abgeleitete Reste, wie Aralkyl, können wie Aryl substituiert sein.

Unter einem gegebenenfalls substituierten araliphatischen Rest werden insbesondere Aralkylreste wie

Arylalkyl, Diarylalkyl, Indanyl oder Fluorenyl verstanden, worin Aryl wie oben definiert ist und in der dort angegebenen Weise substituiert sein kann.

$R^4$ und $R^5$ kann mit den diese tragenden Atomen ein mono-, bi- oder tricyclisches heterocyclisches Ringsystem mit 3 bis 15 Ring-C-Atomen bilden.

Als solche Ringsysteme kommen insbesondere jene aus der folgenden Gruppe in Betracht:
Octahydrocyclopenta[b]pyrrol (D);
Spiro (bicyclo[2.2.2]octan)-2,3′-pyrrolidin] (H);
Pyrrolidin (O); 1,2,3,3a,4,6a-Hexahydrocyclopenta[b]pyrrol (P).

Die in Betracht kommenden heterocyclischen Ringsysteme weisen die folgenden Strukturformeln auf

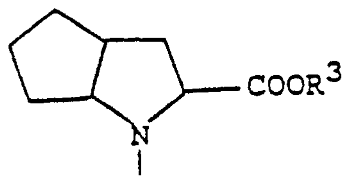

D

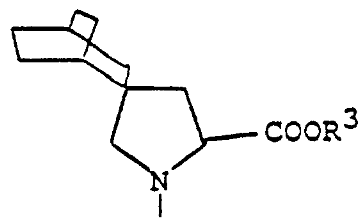

H

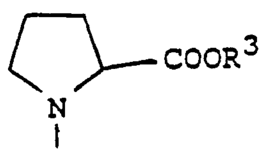

O

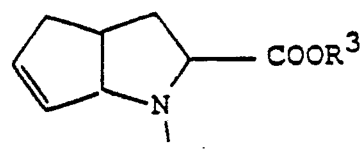

P

Natürlich vorkommende α-Aminosäuren sind beispielsweise Ala, Val, Leu, Ile, Ser, Thr, Asp, Asn, Glu, Gln, Arg, Lys, Hyl, Orn, Cit, Tyr, Phe, Trp und His.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder $(C_1-C_6)$Alkanoyl bevorzugt. Als O-Schutzgruppen für Tyrosin kommen bevorzugt $(C_1-C_6)$-Alkyl, insbesondere Methyl oder Ethyl in Frage.

Die Verbindungen der Formel I weisen asymmetrische Kohlenstoffatome auf und können daher als Enantiomere und Diastereomere auftreten. Die Erfindung umfaßt sowohl die reinen Enantiomeren als auch die Racemate.

Bei Verbindungen der Formel I bzw. II, die mehrere chirale Atome besitzen, kommen alle möglichen Diastereomere als Racemate oder Enantiomere, oder Gemische verschiedener Diastereomere in Betracht.

Als Salze der Verbindungen der Formel I kommen, je nach saurer oder basischer Natur diese Verbindungen, Alkali- oder Erdalkalisalze oder Salze mit physiologisch verträglichen Aminen oder Salze mit anorganischen oder organischen Säuren wie z.B. HCl, HBr, $H_2SO_4$, Maleinsäure, Fumarsäure, Weinsäure, Citronensäure in Frage.

Bevorzugt sind Verbindungen der allgemeinen Formel I, in welcher
n = 2 ist,
R einen aliphatischen Rest mit 4-10 C-Atomen,
einen alicyclischen Rest mit 6 C-Atomen oder
einen aromatischen Rest mit 6 C-Atomen bedeutet,
$R^1$ Methyl oder Benzyl bedeutet,
$R^2$ Wasserstoff,
einen aliphatischen Rest mit 1-18 C-Atomen, Benzyl oder
einen alicyclischen Rest mit 6-10 C-Atomen bedeutet,

$R^3$ Wasserstoff,
einen aliphatischen Rest mit 2-14 C-Atomen oder
einen araliphatischen Rest mit 7-15 C-Atomen bedeutet
und
$R^4$ und $R^5$ zusammen mit den sie tragenden Atomen ein mono-, bi- oder tricyclisches heterocyclisches Ringsystem mit 3-11 C-Atomen bilden
vorausgesetzt, daß

A $R^2$ einen aliphatischen Rest mit 7-18 C-Atomen oder einen alicyclischen Rest mit 7-10 C-Atomen bedeutet und ansonsten n, R, $R^1$, $R^3$, $R^4$ und $R^5$ die oben angegebene Bedeutung besitzen,
oder

B $R^3$ einen aliphatischen Rest mit 7-14 C-Atomen oder einen araliphatischen Rest mit 7-15 C-Atomen bedeutet und ansonsten n, R, $R^1$, $R^2$, $R^4$ und $R^5$ die oben angegebene Bedeutung besitzen,
oder

C R einen aliphatischen Rest mit 9 oder 10 C-Atomen bedeutet und ansonsten n, R , $R^2$, $R^3$, $R^4$ und $R^5$ die oben angegebene Bedeutung besitzen,
und deren physiologisch verträgliche Salze.

Besonders bevorzugt sind Verbindungen der allgemeinen Formel I, in welcher
n = 2 ist,
R n-Butyl, n-Decyl, Cyclohexyl oder Phenyl bedeutet,
$R^1$ Methyl oder Benzyl bedeutet,
$R^2$ Wasserstoff, Methyl, Ethyl, Isobutyl, n-Octyl, n-Octadecyl, Benzyl oder Menthyl bedeutet,
$R^3$ Wasserstoff, Ethyl, n-Octyl, n-Nonyl, 5-Nonyl, n-Decyl, n-Tetradecyl, 2-Octenyl, 2-Octinyl, 3-Octinyl, Benzyl, Benzhydryl oder 3,3-Diphenylpropyl bedeutet und
$R^4$ und $R^5$ zusammen mit den sie tragenden Atomen ein heterocyclisches Ringsystem aus der Reihe Octahydrocyclopenta[b]pyrrol, Spiro[(bicyclo[2.2.2]octan)-2,3'-pyrrolidin], Pyrrolidin oder 1,2,3,3a,4,6a-Hexahydrocyclopenta[b]pyrrol bilden,
vorausgesetzt, daß

A $R^2$ n-Octyl, n-Octadecyl oder Menthyl bedeutet und ansonsten n, R, $R^1$, $R^3$, $R^4$ und $R^5$ die oben angegebene Bedeutung besitzen,
oder

B $R^3$ n-Octyl, n-Nonyl, 5-Nonyl, n-Decyl, n-Tetradecyl, 2-Octenyl, 2-Octinyl, 3-Octinyl, Benzhydryl oder 3,3-Diphenylpropyl bedeutet und ansonsten n, R, $R^1$, $R^2$, $R^4$ und $R^5$ die oben angegebene Bedeutung besitzen
oder

C R n-Decyl bedeutet und ansonsten n, $R^1$, $R^2$, $R^3$, $R^4$ und $R^5$ die angegebene Bedeutung besitzen,
und deren physiologisch verträgliche Salze.

Ganz besonders bevorzugt sind die folgenden Verbindungen der Formel I und deren physiologisch verträgliche Salze.

2-[N-(1S-Ethoxycarbonyl-n-heptyl)-S-alanyl]-(1S,3S,5S)-2-azabicyclo[3.3.0]octan-3-carbonsäure-n-octylester;
2- N-(1S-Ethoxycarbonyl-3-cyclohexylpropyl)-S-alanyl]-(1S,3S,5S)-2-azabicyclo[3.3.0]octan-3-carbonsäure-n-octylester;
2-[N-(1S-Ethoxycarbonyl-n-tridecyl)-S-alanyl]-(1S,3S,5S)-2-azabicyclo[3.3.0]octan-3-carbonsäure-benzylester;
2-[N-(1R-Ethoxycarbonyl-n-tridecyl)-S-alanyl]-(1S,3S,5S)-2-azabicyclo[3.3.0]octan-3-carbonsäure-benzylester;
2-[N-(1S-Ethoxycarbonyl-3-phenylpropyl)-S-alanyl]-(1S,3S,5S)-2-azabicyclo[3.3.0]octan-3-carbonsäure-n-decylester;
2- N-(1S Ethoxycarbonyl-3-phenylpropyl)-S-alanyl]-(1S,3S,5S)-2-azabicyclo[3.3.0]octan-3-carbonsäure-(5-nonyl)-ester;
2-[N-(1S-Isobutyloxycarbonyl-3-phenylpropyl)-S-alanyl]-(1S,3S,5S)-2-azabicyclo[3.3.0]octan-3-carbonsäure-n-octylester;
2- N-(1S-n-Octyloxycarbonyl-3-phenylpropyl)-S-alanyl]-(1S,3S,5S)-2-azabicyclo[3.3.0]octan-3-carbonsäure;
2-[N-(1S-n-Octyloxycarbonyl-3-phenylpropyl)-S-alanyl]-(1S,3S,5S)-2-azabicyclo[3.3.0]octan-3-carbonsäure-ethylester;
2- N-(1S-n-Octyloxycarbonyl-3-phenylpropyl)-S-alanyl]-(1S,3S,5S)-2-azabicyclo[3.3.0]octan-3-carbonsäure-n-octylester;
2-[N-(1S-n-Octadecyloxycarbonyl-3-phenylpropyl)-S-alanyl]-(1S,3S,5S)-2-azabicyclo[3.3.0]octan-3-carbonsäure-benzylester;

2-[N-(1S-n-Octadecyloxycarbonyl-3-phenylpropyl)-S-alanyl]-(1S,3S,5S)-2-azabicyclo[3.3.0]octan-3-carbonsäure-n-octylester;
2-[N-(1S-menthyloxycarbonyl-3-phenylpropyl)-S-alanyl]-(1S,3S,5S)-2-azabicyclo[3.3.0]octan-3-carbonsäure-benzylester;
2-[N-(1S-Menthyloxycarbonyl-3-phenylpropyl)-S-alanyl]-(1S,3S,5S)-2-azabicyclo[3.3.0]octan-3-carbonsäure-benzhydrylester;
2-[N-(1S-Carboxy-3-phenylpropyl)-S-alanyl]-(1S,3S,5S)2-azabicyclo[3.3.0]octan-3-carbonsäure-n-octylester;
2-[N-(1S-Ethoxycarbonyl-3-phenylpropyl)-S-alanyl]-(1S,3S,5S)-2-azabicyclo[3.3.0]-7-octen-3-carbonsäure-n-octylester;
N-[N-(1S-Ethoxycarbonyl-3-phenylpropyl)-S-alanyl]-S-prolin-n-octylester;
2-[N-(1S-Methoxycarbonyl-3-phenylpropyl)-S-alanyl]-(1S,3S, 5S)-2-azabicyclo[3.3.0]octan-3-carbonsäure-n-octylester;
2-[N-(1S-Benzyloxycarbonyl-3-phenylpropyl)-S-alanyl]-(1S,3S, 5S)-2-azabicyclo[3.3.0]octan-3-carbonsäure-n-octylester;
2-[N-(1S-Carboxy-3-phenylpropyl)-S-alanyl]-(1S,3S,5S)-2-azabicyclo[3.3.0]octan-3-carbonsäure-n-decylester.

Die Erfindung betrifft ferner ein Verfahren zur Herstellung von Verbindungen der Formel I, das dadurch gekennzeichnet ist, daß man

a) eine Verbindung der Formel XIV

$$R-(CH_2)_n-\underset{COOQ^2}{\overset{*}{CH}}-NH-\underset{R^1}{\overset{*}{CH}}-\underset{O}{\overset{\|}{C}}-\underset{R^5}{N}-\underset{R^4}{\overset{*}{CH}}-COOQ^3 \qquad (XIV)$$

umsetzt mit einer Verbindung der Formel XV,

Q ═ Y (XV)

wobei in den obengenannten Formeln XIV und XV R, $R^1$, $R^4$, $R^5$ und n die gleiche Bedeutung wie in Formel I haben und

i.

$Q^2$ wie $R^2$ in Formel I definiert ist, aber nicht Wasserstoff bedeutet, oder für eine basisch, sauer oder hydrogenolytisch leicht abspaltbare Carboxyl-Schutzgruppe steht,

$Q^3$ Wasserstoff bedeutet,

Y Hydroxy oder eine nucleophil verdrängbare Abgangsgruppe bedeutet und

Q wie $R^3$ in Formel I definiert ist, aber nicht Wasserstoff bedeutet, oder

Y $\overset{(+)}{=N}$ $\overset{(-)}{=N}$ bedeutet und

Q einen Alkylidenrest mit 1 bis 18 C-Atomen bedeutet, oder

ii.

$Q^2$ Wasserstoff bedeutet,

$Q^3$ wie $R^3$ in Formel I definiert ist, aber nicht Wasserstoff bedeutet, oder für eine basisch, sauer oder hydrogenolytisch leicht abspaltbare Carboxyl-Schutzgruppe steht,

Y Hydroxy oder eine nucleophil verdrängbare Abgangsgruppe bedeutet und

Q wie $R^2$ in Formel I definiert ist, aber nicht Wasserstoff bedeutet, oder

Y $\overset{(+)}{=N}$ $\overset{(-)}{=N}$ bedeutet und

Q einen Alkylidenrest mit 1 bis 18 C-Atomen bedeutet,

oder daß man

b) eine Verbindung der Formel IX

$$R-(CH_2)_n-\underset{COOR^2}{\overset{*}{CH}}-OSO_2CF_3 \qquad (IX)$$

worin R, $R^2$ und n die gleiche Bedeutung wie in Formel I haben, mit einer Verbindung der Formel X

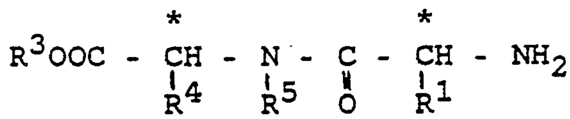 (X)

worin $R^1$, $R^3$, $R^4$ und $R^5$ die gleiche Bedeutung wie in Formel I haben, umsetzt
oder daß man

c) eine Verbindung der Formel XI

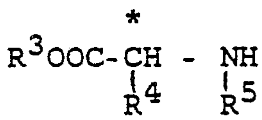 (XI)

worin $R^3$, $R^4$ und $R^5$ die gleiche Bedeutung wie in Formel I haben, mit einer Verbindung der Formel XII

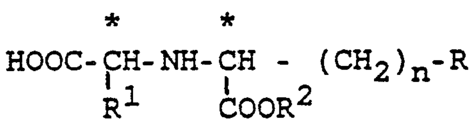 (XII)

worin n, R, $R^1$ und $R^2$ die gleiche Bedeutung wie in Formel I haben, in Analogie zu bekannten Peptidkupplungsverfahren umsetzt,
in der so erhaltenen Verbindung, falls $Q^2$ oder $Q^3$ für eine basisch, sauer oder hydrogenolytisch abspaltbare Schutzgruppe steht, diese Schutzgruppe gegebenenfalls durch Behandeln mit einer Base, einer Säure oder durch Hydrieren abspaltet, und die auf obige Weise erhaltene Verbindung der Formel I gegebenenfalls in ihr physiologisch verträgliches Salz überführt.

Ein Alkylidenrest besitzt die Formel

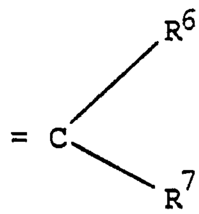

worin $R^6$ und $R^7$ wie unten bei Verfahrensvariante $a_{5)}$ definiert sind.

Gemäß Verfahrensvariante a) setzt man vorzugsweise

$a_{1)}$ eine Verbindung der Formel II

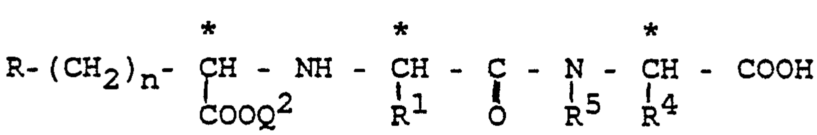 (II)

in der R, $R^1$, $Q^2$, $R^4$, $R^5$ und n die gleiche Bedeutung wie in Formel XIV haben, mit einer Verbindung der Formel III

$R^3$ - OH (III),

in der $R^3$ die gleiche Bedeutung wie in Formel I besitzt, außer der von Wasserstoff, unter Anwendung dem Fachmann vertrauter Veresterungsmethoden (siehe z.B. Buehler, Pearson, Survey of Organic Synthesis, Vol. 1, New York 1970, S. 802-825; Houben-Weyl, Methoden der Organischen Chemie, Band E5, 1985, S. 656-773), z.B. unter saurer Katalyse oder nach Aktivierung der Carbonsäurefunktion von II bzw. der Hydroxyfunktion von III, insbesondere unter den Bedingungen einer Mitsunobu-Reaktion, in einem geeigneten Lösungsmittel bei einer Temperatur bis zum Siedepunkt des Reaktionsgemisches um,
oder man setzt

$a_2$) eine Verbindung der Formel II, in der R, $R^1$, $Q^2$, $R^4$, $R^5$ und n die gleiche Bedeutung wie in Formel XIV haben, mit einer Verbindung der Formel IV

$R^3$ - X (IV),

in der $R^3$ die gleiche Bedeutung wie in Formel I hat, außer der von Wasserstoff, in der X eine Abgangsgruppe, die nucleophil verdrängt werden kann, insbesondere in Cl, Br, J-Atom oder einen Sulfonsäurerest, bedeutet, unter Bedingungen einer nucleophilen Substitution, vorzugsweise in einem polaren organischen Lösungsmittel, wie einem Alkohol, vorzugsweise Methanol, Ethanol, Propanol oder Isopropanol oder einem niederen Keton, vorzugsweise Aceton, Methylethylketon oder Methylisobutylketon oder in Acetonitril, Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumhydrogencarbonat, Kaliumcarbonat, Natriumhydrogencarbonat, Natriumcarbonat, Triethylamin, Pyridin, 1,5-Diazabicyclo[5.4.0]undec-5-en oder 1,5-Diazabicyclo[4.3.0]non-5-en, sowie mit oder ohne Gegenwart eines Alkalihalogenids, vorzugsweise Natriumjodid oder Kaliumjodid, bei einer Temperatur zwischen -50 und +100 °C, vorzugsweise zwischen -20 und +60 °C, um, oder man setzt

$a_3$) eine Verbindung der Formel V

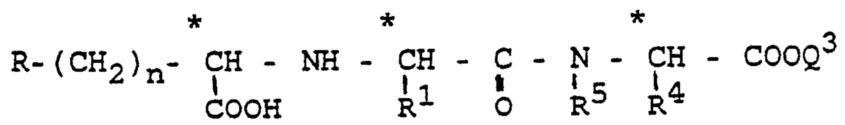 (V)

in der R, $R^1$, $Q^3$, $R^4$, $R^5$ und n die gleiche Bedeutung wie in Formel XIV haben, mit einer Verbindung der Formel VI

$R^2$-OH (VI)

in der $R^2$ die gleiche Bedeutung wie in Formel I besitzt, außer der von Wasserstoff, wie oben unter $a_1$) beschrieben, um, oder man setzt

$a_4$) eine Verbindung der Formel V, in der R, $R^1$, $Q^3$, $R^4$, $R^5$ und n die gleiche Bedeutung wie in Formel XIV haben, mit einer Verbindung der Formel VII

$R^2$-X (VII),

in der $R^2$ die gleiche Bedeutung wie in Formel I besitzt, außer der von Wasserstoff, und in der X die gleiche Bedeutung wie in Formel IV besitzt,wie oben unter $a_2$) beschrieben, um,
oder man setzt

$a_5$) eine Verbindung der Formel II oder V mit einem Diazoalkan der Formel (VIII)

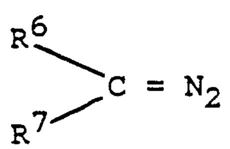 (VIII)

worin $R^6$ einen Rest $C_xH_{2x+1}$ und $R^7$ einen Rest $C_y H_{2y+1}$ bedeuten, x und y jeweils eine ganze Zahl von 0 bis 17 sind und $x + y \leq 17$ ist, in einem inerten organischen Lösungsmittel bei Temperaturen zwischen -80 °C und dem Siedepunkt des Lösungsmittels um.
Die Umsetzung gemäß Variante b) erfolgt z. B. analog der in US-Patent 4525301 beschriebenen Verfahrensweise in einem geeigneten Lösungsmittel bei einer Temperatur bis zum Siedepunkt des Reaktionsgemisches.

Bei Verfahrensweise c) arbeitet man beispielsweise in Analogie zu bekannten Peptidkupplungsverfahren in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphonsäureanhydriden, Dialkylphosphinsäureanhydriden oder N,N-Succinimidoylcarbonat in einem Lösungsmittel wie z. B. Acetonitril oder nach Aktivierung der Verbindungen der Formel XI z.B. durch Umsetzung mit Tetraethyldiphosphit oder nach Aktivierung der Verbindungen der Formel XII in Aktivester (z.B. mit 1-Hydroxybenzotriazol), in gemischte Anhydride (z.B. mit Chlorameisensäureestern), in Azide oder in Carbodiimid-Derivate (vgl.

Schröder, Lübke, The Peptides, Band 1, New York 1965, Seiten 76-136) bei Temperaturen vorzugsweise zwischen -20° C und dem Siedepunkt des Lösungsmittels.

Eine hydrogenolytisch abspaltbare Schutzgruppe spaltet man durch Hydrogenolyse an einem geeigneten Katalysator wie z.B. Palladium auf Aktivkohle bei einem Druck von 0,2 bis 10 bar und einer Temperatur zwischen 0° C und 100° C in einem organischen Lösungsmittel in eine Verbindung der Formel I ($R^3$ = H) ab.

Ein leicht verseifbarer aliphatischer Rest ist, unter Anwendung dem Fachmann vertrauter Verseifungsmethoden (siehe z.B. Houben/Weyl, Methoden der Organischen Chemie, Band E 5/1, Seite 223-255), z.B. durch saure oder alkalische Hydrolyse unter Bildung der freien Carboxylgruppe abspaltbar.

Die Erfindung betrifft weiter Verbindungen der Formel XI, in der

$R^3$ n-Octyl, n-Nonyl, 5-Nonyl, n-Decyl, n-Tetradecyl, 2-Octenyl, 2-Octinyl, 3-Octinyl, Benzyl, Benzhydryl oder 3.3-Diphenylpropyl bedeutet und

$R^4$ und $R^5$ zusammen mit den sie tragenden Atomen ein heterocyclisches Ringsystem aus der Reihe Octahydrocyclopenta[b]pyrrol, Spiro[[bicyclo[2.2.2]octan)-2,3'-pyrrolidin], Pyrrolidin oder 1,2,3,3a,4,6a-Hexahydrocyclopenta[b]-pyrrol bilden, ausgenommen (1S,3S,5S)-2-Azabicyclo[3.3.0]-octan-3-carbonsäure-n-octylester, sowie deren physiologisch verträglichen Salze,

und ein Verfahren zur Herstellung dieser Verbindungen, das dadurch gekennzeichnet ist, daß man eine Verbindung der Formel XI, worin $R^4$ und $R^5$ wie in Formel XI definiert sind und $R^3$ Wasserstoff bedeutet, analog obengenannter Verfahrensvariante a) umsetzt mit einer Verbindung der Formel XV, worin Q wie $R^3$ in Formel XI definiert ist, aber nicht Wasserstoff bedeutet und Y Hydroxy oder eine nucleophil verdrängbare Abgangsgruppe bedeutet.

Verbindungen der Formel II und V sind bekannt (siehe z.B. EP-A 79022, EP-A 105102, EP-A 113880, EP-A 116270, EP-A 74164, EP-A 90362) bzw. auf analogem Wege herstellbar.

Verbindungen der Formel III, IV, VI, VII und VIII sind bekannt und großenteils käuflich.

Verbindungen der Formel IX werden aus Verbindungen der Formel XIII

$$R-(CH_2)_n-\underset{COOR^2}{\overset{*}{C}H}-OH \qquad (XIII)$$

worin R, $R^2$ und n die gleiche Bedeutung wie in Formel I besitzen, durch Überführung der Hydroxylfunktion in die $-OSO_2CF_3$-Gruppe nach gängigen Verfahren erhalten.

Verbindungen der Formel X sind Dipeptide, die aus den einzelnen Aminosäurekomponenten nach an sich bekannten Methoden der Peptidchemie (siehe z.B. Houben-Weyl, Methoden der Organischen Chemie, Bd. XV, Teil II, S. 1-364) aufgebaut werden können.

Verbindungen der Formel XI, XII und XIII sind bekannt bzw. auf analogem Wege wie die bekannten Verbindungen herstellbar.

Die Verbindungen der Formel I und ihre physiologisch verträglichen Salze weisen nootrope, d.h. die cognitive Funktion verbessernde, Wirkung auf. Sie sind daher für die Behandlung cognitiver Dysfunktionen unterschiedlicher Genese, wie sie z.B. bei der Alzheimer'schen Krankheit oder der senilen Demenz auftreten, geeignet. Die nootrope Wirkung der erfindungsgemäßen Verbindungen wurde an Mäusen, die ein Körpergewicht von 20 - 25 g besaßen, im inhibitory (passive) avoidance test (step-through-Modell) geprüft. Eine modifizierte Form der von J. KOPP, Z. BODANECKY und M.E. JARVIK beschriebenen Testmethode wurde von J. BURES, O. BURESOVA und J. HUSTON in "Techniques and Basic Experiments for the Study of Brain and Behavior", Elsevier Scientific Publishers, Amsterdamm (1983) beschrieben.

Entsprechend diesen Literaturangaben wird eine Substanz dann als nootrop wirksam bezeichnet, wenn sie bei den Versuchstieren die mittels eines elektroconvulsiven Schocks erzeugte Amnesie oder die mittels Scopolamin induzierte Amnesie aufzuheben vermag.

Die Versuche wurden nach modifizierten Testmethoden durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß die Scopolamininduzierte Amnesie im inhibitory avoidance-Test sich mit einer MED (minimal effective Dosis) von 0,03 - 30 mg/kg p.o. aufheben läßt. Die Vergleichssubstanz hat eine MED von ca. 500-1000 mg/kg p.o.

Die Erfindung betrifft daher weiter die Anwendung der erfindungsgemäßen Verbindungen bei der Behandlung und Prophylaxe cognitiver Dysfunktionen.

Die Erfindung umfaßt weiterhin die genannten Verbindungen enthaltende Arzneimittel, Verfahren zu

deren Herstellung sowie die Verwendung der erfindungsgemäßen Verbindungen bei der Herstellung von Arzneimitteln, die zur Behandlung und Prophylaxe der vorstehend genannten Krankheiten eingesetzt werden.

In Ausübung der erfindungsgemäßen Methode können die oben beschriebenen Verbindungen der Formel I an Säugern wie Affen, Hunden, Katzen, Ratten, Menschen etc. angewendet werden.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 %, beträgt.

Welche Hilfsstoffe für die gewünschte Arzneimittelformulierung geeignet sind, ist dem Fachmann aufgrund seines Fachwissens geläufig. Neben Lösungsmitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise auf die Schleimhäute (z.B. oral, rektal) oder parenteral (z.B. intravenös oder subcutan) appliziert werden, wobei die orale Applikation bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsform gebracht wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Zur subcutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen oder auch eine Mischung aus den verschiedenen genannten Lösungsmitteln.

Die nachfolgenden Beispiele sollen die erfindungsgemäßen Verbindungen und Verfahren erläutern, ohne die Erfindung auf die hier stellvertretend genannten Substanzen zu beschränken.

## A. Zwischenprodukte

### 1) 2-[N-(1S-Isobutyloxycarbonyl-3-phenylpropyl)-S-alanyl]-(1S,3S,5S)-2-azabicyclo[3.3.0]octan-2-carbonsäure

#### 1a) 2R-Hydroxy-4-phenylbuttersäure-isobutylester

8 g (44 mmol) 2R-Hydroxy-4-phenylbuttersäure werden zusammen mit 100 ml gesättigter Isobutanol/HCl-Lösung 5 Stunden bei Raumtemperatur gerührt, eingeengt, der Rückstand in Ether aufgenommen, mit Wasser, gesättigter $NaHCO_3$-Lösung und erneut Wasser gewaschen, getrocknet und eingeengt.

Ausbeute: 9,35 g (89 %) $[\alpha]_D^{25}$ = -8,1° (c = 1, $CH_3OH$)

Analog werden erhalten

2(RS)-Hydroxytetradecansäureethylester;

2(RS)-Hydroxyoctansäureethylester,

2R-Hydroxy-4-phenylbuttersäuremethylester.

#### 1b) 4-Phenyl-2R-(trifluormethylsulfonyloxy)buttersäureisobutylester

8,8 g (37,6 mmol) Hydroxyester aus Beispiel 1a) und 3,2 ml (40 mmol) Pyridin werden in 200 ml

absolutem Methylenchlorid bei -10° C langsam mit 11,28 g (40 mmol) Trifluormethansulfonsäureanhydrid versetzt, 10 Minuten bei -10° C und 30 Minuten bei Raumtemperatur nachgerührt. Die Reaktionslösung wird eingeengt, der Rückstand in Cyclohexan/Essigester 95/5 gelöst, filtriert und durch Säulenchromatographie an 320 g Kieselgel (Laufmittel Cyclohexan/Essigester 95/5) gereinigt.
Ausbeute: 11,85 g (86 %).

Analog werden erhalten:
2(RS)-(Trifluormethylsulfonyloxy)-octansäureethylester,
2(RS)-(Trifluormethylsulfonyloxy)-tetradecansäureethylester;
4-Phenyl-2R-(trifluormethylsulfonyloxy)-buttersäure-n-octylester;
4-Phenyl-2R-(trifluormethylsulfonyloxy)-buttersäure-n-octadecylester;
4-Phenyl-2R-(trifluormethylsulfonyloxy)-buttersäurementhylester;
4-Phenyl-2(RS)-(trifluormethylsulfonyloxy)-buttersäureethylester;
4-Phenyl-2R-(trifluormethylsulfonyloxy)buttersäuremethylester;
4-Phenyl-2R-(trifluormethylsulfonyloxy)buttersäurebenzylester.

1c) 2-[N-(1S-Isobutyloxycarbonyl-3-phenylpropyl)-S-alanyl](1S,3S,5S)-2-azabicyclo[3.3.0]octan-3-carbonsäurebenzylester

13,85 g (32,2 mmol) N-(S-Alanyl)-(1S,3S,5S)-2-azabicyclo[3.3.0]octan-3-carbonsäurebenzylester-trifluoracetat (siehe Beispiel 3) und 8,7 ml (64,4 mmol) absolutes Triethylamin werden in 85 ml absolutem Methylenchlorid gelöst und bei 0° C eine Lösung von 11,85 g Triflat aus Beispiel 1b) (32,2 mmol) in 85 ml absolutem Methylenchlorid zugetropft. Anschließend wird 4 Stunden bei Raumtemperatur gerührt, die organische Phase dreimal mit Wasser ausgeschüttelt, getrocknet, eingeengt und der Rückstand (13,4 g) an 800 g Kieselgel (Laufmittel: Cyclohexan/Essigester 7:3 und 1:1, dann Toluol/Ethanol) chromatographisch von Nebenprodukten befreit.
Ausbeute 9,65 g (56 %) öliges Produkt
$[\alpha]_D^{25}$ = -39,3° (c = 1, Methanol)

1d) 2-[N-(1S-isobutyloxycarbonyl-3-phenylpropyl)-S-alanyl](1S,3S,5S)-2-azabicyclo[3.3.0]octan-3-carbonsäure

4,0 g (7,5 mmol) Benzylester aus Beispiel 1c) werden in 220 ml Ethanol an 0,6 g Palladium/Kohle (10 %) innerhalb von 30 Minuten bei Raumtemperatur hydriert. Der Katalysator wird abgesaugt und das Filtrat zur Trockne eingeengt.
Ausbeute: 3,32 g (100 %) öliges Produkt
$[\alpha]_D^{25}$ = + 6,8° (c = 1, Methanol).

Analog werden erhalten:
2-[N-(1S-Ethoxycarbonyl-n-heptyl)-S-alanyl]-(1S,3S,5S)-2-azabicyclo[3.3.0]octan-3-carbonsäure;
2- N-(1R-Ethoxycarbonyl-n-heptyl)-S-alanyl]-(1S,3S,5S)-2-azabicyclo[3.3.0]octan-3-carbonsäure;
2-[N-(1S-Ethoxycarbonyl-3-phenylpropyl)-S-alanyl]-(1R,3R,5R)-2-azabicyclo[3.3.0]octan-3-carbonsäure;

2) 2R-Hydroxy-4-phenylbuttersäure-n-octylester

9,0 g (50 mmol) 2R-Hydroxy-4-phenylbuttersäure werden in 250 ml absolutem Dimethylformamid gelöst, 10,0 g (0,1 mmol) Kaliumhydrogencarbonat zugegeben und eine Stunde bei 50° C gerührt. Nach Abkühlen auf Raumtemperatur wird eine Lösung von 15,4 g (80 mmol) 1-Bromoctan in 150 ml absolutem Dimethylformamid zugetropft und drei Stunden bei 50° C nachgerührt. Die Reaktionslösung wird mit 1200 ml Wasser verdünnt, dreimal mit Essigester extrahiert, die vereinigten organischen Phasen zweimal mit Wasser ausgeschüttelt, über Magnesiumsulfat getrocknet, einrotiert und das Rohprodukt (21,3 g) chromatographisch an 480 g Kieselgel (Laufmittel Methylenchlorid/Essigester 100/0, 99:1, 95:5) gereinigt.
Ausbeute 13,4 g (92 %) öliges Produkt
$[\alpha]_D^{25}$ = -6,3° (c = 2, Methanol)

Analog werden erhalten:
2R-Hydroxy-4-phenylbuttersäure-n octadecylester, Schmp. 42-43° C.
2R-Hydroxy-4-phenylbuttersäurebenzylester.

3) 2-(S-Alanyl)-(1S,3S,5S)-2-azabicyclo[3.3.0]-octan-3-carbonsäurebenzylester-trifluoracetat

3a) 2-(N-tert.Butyloxycarbonyl-S-alanyl)-(1S,3S,5S)-2-azabicyclo[3.3.0]octan-3-carbonsäurebenzylester

61,5 g (0,251 mol) (1S,3S,5S)-2-Azabicyclo[3.3.0]octan-3-carbonsäurebenzylester, 47,5 g (0,251 mol) BOC-L-Alanin und 173 ml (1,26 mol) absolutes Triethylamin werden in 1025 ml absolutem Dimethylformamid gelöst, 252 ml einer 50 %igen Propanphosphonsäureanhydridlösung in Dichlormethan bei -5° C zugetropft, 30 Minuten bei -5° C und vier Stunden bei Raumtemperatur nachgerührt. Die Reaktionslösung wird zwischen Wasser und Essigester verteilt, die wäßrige Phase noch einmal mit Essigester extrahiert, die vereinigten organischen Phasen mit gesättigter Natriumbicarbonatlösung, 10 %iger Citronensäurelösung, Wasser und gesättigter Natriumchloridlösung gewaschen, getrocknet und eingeengt.
Ausbeute 93,2 g (89 %) öliges Produkt.

3b) 2-(S-Alanyl)-(1S,3S,5S)-2-azabicyclo[3.3.0]octan-3-carbonsäurebenzylester-trifluoracetat

93,2 g (0,224 mol) BOC-Derivat aus Beispiel 3a) werden bei 0° C mit 235 ml absoluter Trifluoressigsäure übergossen und 2,5 Stunden bei 0° C gerührt. Überschüssige Säure wird im Vakuum bei 25° C abgedampft und der Rückstand aus 1000 ml absolutem Diisopropylether kristalliert. Ausbeute 82,6 g (86 %), Schmp. 148-150° C.

4) N-(1R-Ethoxycarbonyl-3-phenylpropyl)-R-alanin

4a) N-(1R-Ethoxycarbonyl-3-phenylpropyl)-R-alaninbenzylester

Zu einer Lösung von 10,5 g (30 mmol) D-Alaninbenzylester-p-toluolsulfonat in 300 ml absolutem Methylenchlorid werden bei 0° C nacheinander 6,07 g (60 mmol) absolutes Triethylamin und eine Lösung von 10,2 g (30 mmol) 3-Phenyl-2(RS)-(trifluormethylsulfonyloxy)buttersäureethylester zugetropft. Die Reaktionsmischung wird 10 Minuten bei 0° C und 2,5 Stunden bei Raumtemperatur gerührt, mit Methylenchlorid verdünnt, dreimal mit Wasser gewaschen, getrocknet, eingeengt und das Rohprodukt (11,6 g) durch Flash-Chromatographie an 770 g Kieselgel (Laufmittel Cyclohexan/Essigester 9:1) in die Diastereomeren aufgetrennt.
Man erhält nacheinander:
4,78 g (45 %) des RS-Diastereomeren,
$[\alpha]_D^{25}$ = +20,8° (c = 1, Methanol)
3,07 g (29 %) des RR-Diastereomeren,
$[\alpha]_D^{25}$ = +19,7° (c =1, Methanol).
Analog werden erhalten:
N-(1S-Ethoxycarbonyl-3-phenylpropyl)-S-alanin-benzylester,
$[\alpha]_D^{25}$ = -18,4° (c = 1, Methanol);
N-(1R-Ethoxycarbonyl-3-phenylpropyl)-S-alanin-benzylester,
$[\alpha]_D^{25}$ = -21,7° (c = 1, Methanol);
N-(1S-Ethoxycarbonyl-n-heptyl)-S-alanin-benzylester,
$[\alpha]_D^{25}$ = -31,2° (c = 1, Methanol);
N-(1R-Ethoxycarbonyl-n-heptyl)-S-alanin-benzylester;
N-(1S-Ethoxycarbonyl-n-tridecyl)-S-phenylalanin-benzylester;
N-(1(RS)-Ethoxycarbonyl-n-tridecyl)-S-phenylalanin-benzylester;

4b) N-(1R-Ethoxycarbonyl-3-phenylpropyl)-R-alanin

3,0 g (8,12 mmol) RR-Diastereomeres aus Beispiel 4a) werden in 75 ml Ethanol gelöst und bei Raumtemperatur während einer Stunde an 250 mg Palladium/Kohle (10 %) hydriert. Der Katalysator wird abgesaugt, das Filtrat eingeengt.
Ausbeute 2,2 g (96 %) farblose Kristalle, Schmp. 146-148° C.

Auf analogem Wege werden erhalten:
N-(1S-Ethoxycarbonyl-3-phenylpropyl)-R-alanin, Schmp. 134-136 °C;
N-(1R-Ethoxycarbonyl-3-phenylpropyl)-S-alanin, Schmp. 133-135 °C;
N-(1S-Ethoxycarbonyl-3-phenylpropyl)-S-alanin, Schmp. 148-150 °C;
N-(1S-Ethoxycarbonyl-n-heptyl)-S-alanin,
$[\alpha]_D^{23}$ = +9,2° (c = 1, Methanol);
N-(1R-Ethoxycarbonyl-n-heptyl)-S-alanin, Schmp. 122-124 °C;
N-(1S-Ethoxycarbonyl-n-tridecyl)-S-phenylalanin, Schmp. 116-118 °C;
N-(1(RS)-Ethoxycarbonyl-n-tridecyl)-S-phenylalanin, Schmp. 87-90 °C;

5) 2-[N-(1S-Ethoxycarbonyl-n-heptyl)-S-alanyl]-(1S,3S,5S)-2-azabicyclo[3.3.0]octan-3-carbonsäurebenzylester

Zu einer Lösung von 2,3 g (8,9 mmol) N-(1S-Ethoxycarbonyl-n-heptyl)-S-alanin und 2,8 g (10,0 mmol) (1S,3S,5S)-2-Azabicyclo[3.3.0]octan-3-carbonsäurebenzylester in 110 ml absolutem Dimethylformamid werden bei -8 °C nacheinander 6,0 ml absolutes Triethylamin und 8,2 ml einer 50 %igen Lösung von Propanphosphonsäureanhydrid in Methylenchlorid zugetropft. Die Reaktionslösung wird über das Wochenende bei Raumtemperatur stehengelassen, mit Essigester verdünnt, mit gesättigter Natriumcarbonatlösung, 10 %iger Citronensäurelösung und gesättigter Natriumchloridlösung gewaschen, getrocknet, eingeengt und der Rückstand (3,9 g) durch Chromatographie an Kieselgel (Laufmittel Cyclohexan/Essigester 7:3) gereinigt.
Ausbeute 1,2 g (28 %) öliges Produkt,
$[\alpha]_D^{25}$ = -46,6° (c = 1, Methanol).

Auf analogem Wege werden erhalten:
2-[N-(1R-Ethoxycarbonyl-n-heptyl)-S-alanyl]-(1S,3S,5S)-2-azabicyclo[3.3.0]octan-3-carbonsäurebenzylester;
$[\alpha]_D^{25}$ = -32,5° (c = 0,79, Methanol),
2-[N-(1S-Ethoxycarbonyl-3-phenylpropyl)-S-alanyl]-(1R,3R,5R)-2-azabicyclo[3.3.0]octan-3-carbonsäurebenzylester,
$[\alpha]_D^{25}$ = -1,9° (c = 1, Methanol).

6) (1S,3S,5S)-2-Azabicyclo[3.3.0]octan-3-carbonsäure-n-octylester

6a) 2-tert.Butyloxycarbonyl-(1S,3S,5S)-2-azabicyclo[3.3.0]octan-3-carbonsäurebenzylester

Zu einer Lösung von 40,0 g (0,163 mol) (1S,3S,5S)-2-Azabicyclo[3.3.0]octan-3-carbonsäurebenzylester und 23,4 ml (0,169 mol) absolutem Triethylamin in 300 ml absolutem Methylenchlorid wird bei 0 °C eine Lösung von 39,2 g (0,18 mmol) Di-tert.butyldicarbonat in 60 ml absolutem Methylenchlorid langsam zugetropft, 15 Minuten bei 0 °C und eine Stunde bei Raumtemperatur nachgerührt. Die Reaktionslösung wird mit 10 %iger Citronensäurelösung, gesättigter Natriumbicarbonatlösung und Wasser gewaschen, getrocknet und eingeengt.
Ausbeute: 55.6 g öliges Produkt,
$[\alpha]_D^{25}$ = -1,2° (c = 2, Methanol).

6b) 2-tert.Butyloxycarbonyl-(1S,3S,5S)-2-azabicyclo[3.3.0]octan-3-carbonsäure

55,6 g (0,161 mol) Benzylester aus Beispiel 6a) werden in 2 l Ethanol bei Raumtemperatur an 4 g Palladium/Kohle (10%) während 2,5 Stunden hydriert. Der Katalysator wird abgesaugt und das Filtrat eingeengt.
Ausbeute 37,3 g (90 %);
$[\alpha]_D^{25}$ = +22,7° (c = 1, Methanol).

6c) 2-tert.Butyloxycarbonyl-(1S,3S,5S)-2-azabicyclo[3.3.0]octan-3-carbonsäure-n-octylester

32,3 g (0,127 mol) Säure aus Beispiel 6b) und 25,3 g (0,253 mol) Kaliumhydrogencarbonat werden in

500 ml Dimethylformamid 1,5 Stunden bei 40° C gerührt. Nach Abkühlen werden 48,9 g (0,253 mol) 1-Bromoctan zugetropft und über Nacht bei Raumtemperatur gerührt. Die Reaktionsmischung wird auf Wasser gegeben, dreimal mit Essigester extrahiert, die vereinigten organischen Phasen mit gesättigter Natriumhydrogencarbonatlösung und Wasser gewaschen, getrocknet, eingeengt und das Rohprodukt (44,3 g) durch Flash-Chromatographie an Kieselgel (900 g, Laufmittel Toluol/Ethanol 95:5 bzw. 99,5:0,5) in zwei Portionen gereinigt.
Ausbeute: 35,4 g (76 %) öliges Produkt,
$[\alpha]_D^{25}$ = +5,7° (c = 1, Methanol).

Auf analogem Wege werden erhalten:
2-Tert.butyloxycarbonyl-(1RS,3RS,5RS)-2-azabicyclo[3.3.0]-7-octen-3-carbonsäure-n-octylester;
N-tert.Butyloxycarbonyl-S-prolin-n-octylester.

6d) (1S,3S,5S)-2-Azabicyclo[3.3.0]octan-3-carbonsäure-n-octylester

2,6 g (7,0 mol) BOC-Verbindung aus Beispiel 6c) werden bei 0° C mit 9 ml Trifluoressigsäure 1,5 Stunden lang gerührt. Die überschüssige Säure wird im Vakuum abgedampft, der Rückstand in Wasser aufgenommen, mit Natriumbicarbonat basisch gestellt, mit Essigester extrahiert, die organische Phase noch einmal mit Wasser gewaschen, getrocknet eingeengt und das Produkt rasch weiter umgesetzt. Ausbeute: 1,8 g (95 %) öliges Produkt.

Auf analogem Wege werden erhalten:
(1RS,3RS,5RS)-2-Azabicyclo[3.3.0]-7-octen-3-carbonsäure-n-octylester;
S-Prolin-n-octylester.

7) 2-tert.Butyloxycarbonyl-(1RS,3RS,5RS)-2-azabicyclo[3.3.0]-7-octen-3-carbonsäure

4,0 g (26,1 mmol) (1RS,3RS,5RS)-2-Azabicyclo[3.3.0]-7-octen-3-carbonsäure (cis-endo-Racemat) werden unter Eiskühlung in einem Gemisch aus 78 ml Dioxan/Wasser 2:1 und 26,1 ml 1 N Natronlauge mit 6,26 g (28,7 mmol) Di-tert.- butyldicarbonat versetzt und eine Stunde bei Raumtemperatur gerührt, wobei der pH-Wert durch Zugabe von 1 N Natronlauge ständig bei pH 9 gehalten wird.
Die Reaktionslösung wird eingeengt, der Rückstand in Wasser gelöst, mit Essigester überschichtet und mit gesättigter Kaliumhydrogensulfatlösung auf pH 2 gestellt. Die Phasen werden getrennt, die wäßrige Phase noch zweimal mit Essigester extrahiert und die vereinigten organischen Phasen mit Wasser und gesättigter Natriumchloridlösung gewaschen, getrocknet und eingeengt.
Ausbeute: 6,3 g (96 %).

8) Methansulfonsäure-3-octinylester

Zu einer Lösung von 7,56 g (60 mmol) 3-Octin-1-ol und 12,45 ml (90 mmol) Triethylamin in 225 ml Methylenchlorid werden bei 10° C innerhalb von 30 Minuten 7,47 g (65 mmol) Methansulfonsäurechlorid zugetropft und eine Stunde nachgerührt. Die Reaktionslösung wird mit Wasser, gesättigter Natriumbicarbonatlösung und erneut mit Wasser gewaschen, getrocknet und eingeengt
Ausbeute 11,9 g (97 %) öliges Produkt.

B. Endprodukte

Beispiel 9:

2- N-(1S-Ethoxycarbonyl-3-phenylpropyl)-S-alanyl]-(1S,3S,5S)-2-azabicyclo[3.3.0]octan-3-carbonsäure-n-octylester

2,07 g (5 mmol) 2-[N-(1S-Ethoxycarbonyl-3-phenylpropyl)-S-alanyl]-(1S,3S,5S)-2-azabicyclo[3.3.0]-octan-3-carbonsäure (Ramipril) und 0,50 g (5 mmol) Kaliumhydrogencarbonat werden in 25 ml Dimethylformamid 1,5 Stunden bei 40° C gerührt, nach Abkühlen auf Raumtemperatur eine Lösung von 1,16 g (6

mmol) 1-Bromoctan in 20 ml Dimethylformamid zugetropft und über Nacht bei Raumtemperatur gerührt. Durch Zugabe von 0,1 N HCl wird auf pH 6 gestellt, mit Wasser verdünnt, dreimal mit Methylenchlorid extrahiert und die vereinigten organischen Phasen getrocknet, eingeengt und durch Säulenchromatographie an 120 g Kieselgel (Laufmittel Toluol/Ethanol 95:5) gereinigt.
Ausbeute: 2,35 g (89 %) öliges Produkt;
$[\alpha]_D^{25}$ = -23,9° (c = 1, Methanol).

Beispiel 10:

2-[N-(1S-Ethoxycarbonyl-3-phenylpropyl)-S-alanyl]-(1S,3S,5S)-2-azabicyclo[3.3.0]octan-3-carbonsäure-n-octylester-hydrogenmaleinat

528 mg (1 mmol) eines nach Beispiel 9 gewonnenen Amins werden in 20 ml Ether gelöst und mit einer Lösung von 116 mg (1 mmol) Maleinsäure in 4 ml Aceton versetzt. Die Lösungsmittel werden abgedampft und der Rückstand mit Diisopropylether kristallisiert.
Ausbeute: 0,51 g (79 %) farblose Kristalle, Schmp. 89-90° C.

Beispiel 11:

2-[N-(1S-Ethoxycarbonyl-3-phenylpropyl)-S-alanyl]-(1S,3S,5S)-2-azabicyclo[3.3.0]octan-3-carbonsäure-2-octinylester-hydrogenmaleinat

2,08 g (5 mmol) 2-[N-(1S-Ethoxycarbonyl-3-phenypropyl)-S-alanyl]-(1S,3S,5S)-2-azabicyclo[3.3.0]octan-3-carbonsäure (Ramipril) und 1,00 g (10 mmol) Kaliumhydrogencarbonat werden in 25 ml Dimethylformamid 1,5 Stunden bei 40° C gerührt, auf 0° C abgekühlt und eine Lösung von 2,3 g (12 mmol) E-1-Brom-2-octen in 20 ml Dimethylformamid zugetropft. Die Reaktionslösung wird 4 Stunden bei 0° C gerührt, auf 500 ml Wasser gegeben, dreimal mit Essigester extrahiert, die vereinigten Extrakte zweimal mit gesättigter Natriumbicarbonatlösung und dreimal mit Wasser gewaschen, getrocknet, eingeengt und das Rohprodukt (3,4 g) durch Flash-Chromatographie an 125 g Kieselgel (Laufmittel Cyclohexan/Essigester 8:2 und 1:1) gereinigt. Man erhält 1,93 g (73 %) eines öligen Produktes, das analog Beispiel 10 ins Hydrogenmaleinat übergeführt wird. Ausbeute 2,0 g farblose Kristalle, Schmp. 81-84° C.

Beispiel 12:

2-[N-(1S-Ethoxycarbonyl-3-phenylpropyl)-S-alanyl]-(1S,3S,5S)-2-azabicyclo[3.3.0]octan-3-carbonsäure-3-octinylester-hydrogenmaleinat

4.9 g (11,8 mmol) 2-[N-(1S-Ethoxycarbonyl-3-phenylpropyl)-S-alanyl]-(1S,3S,5S)-2-azabicyclo[3.3.0]-octan-3-carbonsäure (Ramipril) und 2,4 g (23,6 mmol) Kaliumhydrogencarbonat werden in 90 ml Dimethylformamid 2 Stunden bei 40° C gerührt, dann eine Lösung von 2,41 g (11,8 mmol) Mesylat aus Beispiel 8 in 30 ml Dimethylformamid zugegeben und weitere 9 Stunden bei 40° C gerührt. Die Reaktionslösung wird mit 250 ml Wasser verdünnt, dreimal mit Essigester extrahiert, die vereinigten organischen Phasen mit gesättigter Natriumbicarbonatlösung und mit Wasser gewaschen, getrocknet, eingeengt und das Rohprodukt (5,6 g) durch Chromatographie an 200 g Kieselgel (Laufmittel Toluol/Ethanol 99:1) gereinigt. Man erhält 3,45 g (56 %) öliges Produkt, von dem 1,3 g analog Beispiel 10 ins Hydrogenmaleinat überführt werden.
Ausbeute: 0,8 g farblose Kristalle, Schmp. 68-70° C.

Beispiel 13:

2-[N-(1S-n-Octyloxycarbonyl-3-phenylpropyl)-S-alanyl]-(1S,3S,5S)-2-azabicyclo 3.3.0]octan-3-carbonsäure-ethylester-hydrogenmaleinat

1,43 g (2,8 mmol) Carbonsäure aus Beispiel 19 werden in 25 ml ethanolischer Salzsäure bei Raumtemperatur gerührt. Nach 5 Tagen werden weitere 25 ml ethanolische Salzsäure zugegeben, über

Nacht gerührt, eingeengt, der Rückstand in Essigester aufgenommen, dreimal mit gesättigter Natriumbicarbonatlösung und einmal mit Wasser gewaschen, getrocknet, eingeengt und das Rohprodukt (1,16 g) durch Flash-chromatographie an 80 g Kieselgel (Laufmittel Toluol/Ethanol 99:1) gereinigt. Man erhält 0,62 g (42 %) öliges Produkt, das analog Beispiel 10 ins Hydrogenmaleinat übergeführt wird.
Ausbeute: 0,50 g farblose Kristalle, Schmp. 84-86° C.

Beispiel 14:

2-[N-(1S-Ethoxycarbonyl-3-phenylpropyl)-S-alanyl]-(1S,3S,5S)-2-azabicyclo[3.3.0]octan-3-carbonsäure-5-nonylester-hydrogenmaleinat

Zu einer Lösung von 1,97 g (7,5 mol) Triphenylphosphin und 0,72 g (5 mmol) 5-Nonanol in 100 ml absolutem Tetrahydrofuran wird bei 0° C eine Lösung von 1,31 g (7,5 mmol) Azodicarbonsäurediethylester in 10 ml absolutem Tetrahydrofuran zugetropft, 10 Minuten nachgerührt, dann bei 0° C eine Lösung von 2,08 g (5 mmol) 2-[N-(1S-Ethoxycarbonyl-3-phenylpropyl)-S-alanyl]-(1S,3S,5S)-2-azabicyclo[3.3.0]octan-3-carbonsäure (Ramipril) in 25 ml absolutem Tetrahydrofuran zugegeben, eine Stunde bei 0° C und über Nacht bei Raumtemperatur gerührt. Die Reaktionslösung wird eingeengt, in Essigester aufgenommen, zweimal mit 2 N Natronlauge und einmal mit Wasser gewaschen, getrocknet, eingeengt und das Rohprodukt (5,0 g) durch zweifache Flash-Chromatographie an 200 g Kieselgel (Laufmittel a) Toluol/Ethanol 99:1, b) Methylenchlorid/Essigester 9:1) gereinigt. Das so erhaltene Produkt (1,74 g, 64 %) wird analog Beispiel 10 ins Hydrogenmaleinat übergeführt.
Ausbeute: 1,6 g (49 %); Schmp. 103-105° C.

Beispiel 15:

2-[N-(1S-menthyloxycarbonyl-3-phenylpropyl)-S-alanyl]-(1S,3S,5S)-2-azabicyclo[3.3.0]octan-3-carbonsäurebenzhydrylester

Zu einer Suspension von 2,95 g Nickelperoxid-hydrat in 12 ml Ether wird bei Raumtemperatur eine Lösung von 0,59 g (3 mmol) Benzophenonhydrazon in 12 ml Ether zugetropft, eine Stunde nachgerührt, die violette Lösung über Celite abgesaugt und eingeengt. Die so erhaltenen 3 mmol Diphenyldiazomethan werden in 32 ml absolutem Aceton gelöst und unter Eiskühlung zu einer Lösung von 1,31 g (2,5 mmol) 2-[N-(1S-Menthyloxycarbonyl-3-phenylpropyl)-S-alanyl]-(1S,3S,5S)-2-azabicyclo[3.3.0]octan-3-carbonsäure (siehe Beispiel 45) in 32 ml absolutem Aceton zugetropft. Anschließend wird 38 Stunden bei Raumtemperatur nachgerührt, eingeengt und das Rohprodukt durch Säulenchromatographie an Kieselgel (Laufmittel Toluol/Ethanol 99,5:0,5 bzw. Cyclohexan/Essigester 8:2) gereinigt.
Ausbeute: 1,63 g (95 %) öliges Produkt;
$[\alpha]_D^{25}$ = -57,9° (c = 1, Methanol).

Beispiel 16:

2-[N-(3-Cyclohexyl-1S-ethoxycarbonyl-propyl)-S-alanyl]-(1S,3S,5S)-2-azabicyclo[3.3.0]octan-3-carbonsäure-n-octylester

1,8 g (6,7 mmol) Octylester aus Beispiel 6d), 1,92 g (6,7 mmol) N-(3-Cyclohexyl-1S-ethoxycarbonyl-propyl)-S-alanin und 4,6 ml absolutes Triethylamin werden in 30 ml absolutem Dimethylformamid gelöst, auf -5° C abgekühlt und langsam 6,7 ml einer 50 %igen Lösung von Propanphosphonsäureanhydrid in Methylenchlorid zugetropft. Die Reaktionslösung wird über Nacht bei Raumtemperatur gerührt, auf 200 ml Wasser gegeben, dreimal mit Essigester extrahiert, die vereinigten organischen Phasen mit Wasser, 10 %iger Citronensäurelösung, gesättigter Natriumcarbonatlösung und gesättigter Natriumchloridlösung gewaschen, getrocknet, eingeengt und das Rohprodukt (3,1 g) durch Flash-chromatographie an 120 g Kieselgel (Laufmittel Toluol/Ethanol 199:1) gereinigt.
Ausbeute: 2,48 g (69 %) farbloses Öl,
$[\alpha]_D^{25}$ = -26,4° (c = 1, Methanol).

Beispiel 17:

2- N-(1S-Ethoxycarbonyl-n-tridecyl)-S-phenylalanyl]-(1S,3S,5S)-2-azabicyclo[3.3.0]octan-3-carbonsäurebenzylester

Zu einer Lösung von 2,1 g (5 mmol) N-(1S-Ethoxycarbonyl-n-tridecyl)-S-phenylalanin und 1,4 g (5 mmol) (1S,3S,5S)-2-azabicyclo[3.3.0]octan-3-carbonsäurebenzylester in 80 ml absolutem Dimethylformamid werden nacheinander 3,5 ml (25 mmol) absolutes Triethylamin und 5,0 ml einer 50 %igen Lösung von Propanphosphonsäureanhydrid in Methylenchlorid zugetropft und die Reaktionslösung über Nacht bei Raumtemperatur gerührt. Dann wird auf Wasser gegeben, mehrfach mit Essigester extrahiert, die vereinigten Extrakte mit Wasser, 10 % Citronensäurelösung, gesättigter Natriumbicarbonatlösung und gesättigter Natriumchloridlösung gewaschen, getrocknet, eingeengt und das Rohprodukt (3,15 g) durch Flash-Chromatographie an Kieselgel (Laufmittel Cyclohexan/Essigester 7:3) gereinigt.
Ausbeute: 2,76 g (85 %) öliges Produkt
$[\alpha]_D^{20}$ = -8,0° (c = 0,97, Ethanol).

Beispiel 18:

2-[N-(1S-n-Octyloxycarbonyl-3-phenylpropyl)-S-alanyl]-(1S,3S,5S)-2-azabicyclo[3.3.0]octan-3-carbonsäurebenzylester

Zu einer Lösung von 9,3 g (21,7 mmol) Trifluoracetat aus Beispiel 3b) in 100 ml absolutem Methylenchlorid werden bei 0°C nacheinander 6,0 ml (43,4 mmol) absolutes Triethylamin und 9,2 g 4-phenyl-2R-(trifluormethansulfonyloxy)buttersäure-n-octylester, gelöst in 20 ml absolutem Methylenchlorid, zugetropft. Man läßt auf Raumtemperatur kommen, rührt 2,5 Stunden nach, extrahiert dreimal mit Wasser, trocknet, engt ein und reinigt das Rohprodukt (11,4 g) durch Flash-Chromatographie an 450 g Kieselgel (Laufmittel Cyclohexan/Essigester 9:1, 8:2, 7:3).
Ausbeute: 6,95 g (54 %) öliges Produkt.
$[\alpha]_D^{25}$ = -35,1° (c = 1, Methanol).

Beispiel 19:

tert.Butylammonium-[2-[N-(1S-n-octyloxycarbonyl-3-phenylpropyl)-S-alanyl]-(1S,3S,5S)-2-azabicyclo[3.3.0] octan-3-carboxylat]

5,45 g (9,2 mmol) Benzylester aus Beispiel 18 werden in 300 ml Ethanol bei Raumtemperatur an 1 g Palladium/Kohle (10 %) während 20 Minuten hydriert. Nach Absaugen des Katalysators und Einengen erhält man 4,1 g (89 %) 2-[N-(1S-n-Octyloxycarbonyl-3-phenylpropyl)-S-alanyl]-(1S,3S,5S)-2-azabicyclo[3.3.0]-octan-3-carbonsäure.

1,3 g dieser Säure werden in Ethanol mit 190 mg tert. Butylamin versetzt, das Lösungsmittel abgedampft und der Rückstand mit Diisopropylether kristallisiert.
Ausbeute 1,27 g (86 %) farblose Kristalle.
Schmp. 141-143°C

Beispiel 20:

2-[N-(1S-Carboxy-3-phenylpropyl)-S-alanyl]-(1S,3S,5S)-2-azabicyclo[3.3.0]octan-3-carbonsäure-n-octylester

2,65 g (5 mmol) Ethylester aus Beispiel 9 werden in 18 ml Tetrahydrofuran gelöst, 7,5 ml 1 N Natronlauge zugegeben und 48 Std. bei Raumtemperatur gerührt. Man neutralisiert durch Zugabe von 7,5 ml 1 N Salzsäure. Das Reaktionsgemisch wird eingeengt, der Rückstand in Wasser suspendiert, zweimal mit Essigester extrahiert, die vereinigten organischen Phasen mit gesättigter Natriumchloridlösung gewaschen, getrocknet, eingeengt und das Rohprodukt (2,05 g) durch Chromatographie an 80 g Kieselgel (Toluol/Ethanol 9:1) gereinigt. Das so erhaltene Produkt (1,15 g; 46 %) wird in 50 ml Petrolether verrieben, kaltgestellt, abgesaugt und getrocknet.

Ausbeute: 0,83 g farblose Kristalle; Schmp. 56-61 °C.

Unter Verwendung geeigneter Ausgangsmaterialien und unter Anwendung der in den Beispielen 9-20 beschriebenen Verfahren werden außerdem die folgenden erfindungsgemäßen Verbindungen erhalten:

Beispiel 21:

2-[N-(1S-Ethoxycarbonyl-n-heptyl)-S-alanyl]-(1S,3S,5S)-2-azabicyclo[3.3.0]octan-3-carbonsäure-n-octylester

Beispiel 22:

2-[N-(1S-Ethoxycarbonyl-3-phenylpropyl)-S-alanyl]-(1S,3S,5S)-2-azabicyclo[3.3.0]octan-3-carbonsäure-n-decylester; $[\alpha]_D^{25}$ = -25,8° (c = 1, Methanol).

Beispiel 23:

2-[N-(1S-Ethoxycarbonyl-3-phenylpropyl)-S-alanyl]-(1S,3S,5S)-2-azabicyclo[3.3.0]octan-3-carbonsäure-n-tetradecylester; $[\alpha]_D^{25}$ = -19,8° (c = 1, Methanol).

Beispiel 24:

2-[N-(1S-Ethoxycarbonyl-3-phenylpropyl)-S-alanyl]-(1S,3S,5S)-2-azabicyclo[3.3.0]octan-3-carbonsäure-(2-octinyl)ester-hydrogenmaleinat; Schmp. 70-72 °C.

Beispiel 25:

2-[N-(1S-isobutyloxycarbonyl-3-phenylpropyl)-s-alanyl]-(1S,3S,5S)-2-azabicyclo[3.3.0]octan-3-carbonsäure-n-octylester; $[\alpha]_D^{25}$ = -23,7° (c = 1, Methanol).

Beispiel 26:

2-[N-(1S-n-Octyloxycarbonyl-3-phenylpropyl)-S-alanyl]-(1S,3S,5S)-2-azabicyclo[3.3.0]octan-3-carbonsäure-n-octylester; $[\alpha]_D^{25}$ = -18,6° (c = 1, Methanol).

Beispiel 27:

2-[N-(1S-n-octadecycloxycarbonyl-3-phenylpropyl)-S-alanyl]-(1S,3S,5S)-2-azabicyclo[3.3.0]octan-3-carbonsäure-n-octylester; $[\alpha]_D^{25}$ = -15,2° (c = 1, Methanol).

Beispiel 28:

1′-[N-(1S-Ethoxycarbonyl-3-phenylpropyl)-S-alanyl]-spiro[bicyclo[2.2.2]octan-2,3′-pyrrolidin]-5′S-carbonsäure-n-octylester;

Beispiel 29:

1′- N-(1S-Ethoxycarbonyl-3-phenylpropyl)-S-alanyl]-spiro [bicyclo[2.2.2]octan-2,3′-pyrrolidin]-5′S-carbonsäure-n-nonylester-hydrogenmaleinat; Schmp. 110 °C.

Beispiel 30:

1′-[N-(1S-Ethoxycarbonyl-3-phenylpropyl)-S-alanyl]-spiro bicyclo[2.2.2]octan-2,3′-pyrrolidin]-5's-carbonsäure-n-decylester-hydrogenmaleinat; Schmp. 96 °C.

Beispiel 31:

2-[N-(1S-Ethoxycarbonyl-3-phenylpropyl)-s-alanyl]-(1R,3R 5R)-2-azabicyclo[3.3.0]octan-3-carbonsäure-n-octylester; $[\alpha]_D^{25}$ = -7,0° (c = 1, Methanol).

Beispiel 32:

2-[N-(1S-Ethoxycarbonyl-3-phenylpropyl)-R-alanyl]-(1S,3S,5S)-2-azabicyclo[3.3.0]octan-3-carbonsäure-n-octylester; $[\alpha]_D^{25}$ = +18,0° (c = 1, Methanol).

Beispiel 33:

2-[N-(1R-Ethoxycarbonyl-3-phenylpropyl)-R-alanyl]-(1S,3S,5S)-2-azabicyclo[3.3.0]octan-3-carbonsäure-n-octylester; $[\alpha]_D^{25}$ = +9,4° (c = 1, Methanol).

Beispiel 34:

2-[N-(1S-Ethoxycarbonyl-3-phenylpropyl)-S-alanyl]-(1S,3S,5S)-2-azabicyclo[3.3.0]-7-octen-3-carbonsäure-n-octylester; $[\alpha]_D^{25}$ = + 21,2° (c = 1, Methanol).

Beispiel 35:

2-[N-(1S-Ethoxycarbonyl-3-phenylpropyl)-S-alanyl]-(1R,3R,5R)-2-azabicyclo[3.3.0]-7-octen-3-carbonsäure-n-octylester; $[\alpha]_D^{25}$ = -51,4° (c = 1, Methanol).

Beispiel 36:

N-[N′-(1S-Ethoxycarbonyl-3-phenylpropyl)-S-alanyl]-S-prolin-n-octylester-hydrogenmaleinat; Schmp. 105-107° C.

Beispiel 37:

2-[N-(1S-Ethoxycarbonyl-n-tridecyl)-s-phenylalanyl]-(1S,3S,5S)-2-azabicyclo[3.3.0]octan-3-carbonsäure; $[\alpha]_D^{20}$ = +27,2° (c = 1, Methanol).

Beispiel 38:

2-[N-(1S-Ethoxycarbonyl-n-tridecyl)-S-alanyl]-(1S,3S,5S)-2-azabicyclo[3.3.0]octan-3-carbonsäure-benzylester;

Beispiel 39:

2-[N-(1R-Ethoxycarbonyl-n-tridecyl)-S-alanyl]-(1S,3S,5S)-2-azabicyclo[3.3.0]octan-3-carbonsäure-benzylester;

Beispiel 40

2-[N-(1S-Octadecyloxycarbonyl-3-phenylpropyl)-S-alanyl]-(1S,3S,5S)-2-azabicyclo[3.3.0]octan-3-carbonsäurebenzylester; $[\alpha]_D^{25}$ = -28,6° (c = 1, Methanol)

Beispiel 41

2-[N-(1S-Menthyloxycarbonyl-3-phenylpropyl)-S-alanyl]-(1S,3S,5S)-2-azabicyclo[3.3.0]octan-3-carbonsäurebenzylester; $[\alpha]_D^{25}$ = -63,2° (c = 1, Methanol).

Beispiel 42

2-[N-(1S-Ethoxycarbonyl-3-phenylpropyl)-S-alanyl]-(1S,3S,5S)-2-azabicyclo[3.3.0]octan-3-carbonsäure- (3,3-diphenyl-n-propyl)ester-hydrogenmaleinat; Schmp. 122-124° C.

Beispiel 43

1′-[N-(1S-Ethoxycarbonyl-3-phenylpropyl)-S-alanyl]-spiro[bicyclo[2.2.2]octan-2,3′-pyrrolidin]-5′S-carbonsäure (5-nonyl)ester-hydrogenmaleinat,
Schmp. 121 °C

Beispiel 44

tert.Butylammonium[2-[N-(1S-octadecyloxycarbonyl-3-phenylpropyl)-S-alanyl]-(1S,3S,5S)-2-azabicyclo[3.3.0] octan-3-carboxylat]; Schmp. 133-135 °C.

Beispiel 45

tert.Butylammonium[2-[N-(1S-Menthyloxycarbonyl-3-phenylpropyl)-S-alanyl]-(1S,3S,5S)-2-azabicyclo[3.3.0]-octan-3-carboxylat]; Schmp. 164-167 °C.

Beispiel 46

2- N-(1R-Ethoxycarbonyl-3-phenylpropyl)-S-alanyl]-(1S,3S, 5S)-2-azabicyclo[3.3.0]octan-3-carbonsäure-n-octylester $[\alpha]_D^{25}$ = - 30,8° (c = 1, Methanol).

Beispiel 47

2-[N-(1R-Ethoxycarbonyl-3-phenylpropyl)-R-alanyl]-(1R,3R, 5R)-2-azabicyclo[3.3.0]octan-3-carbonsäure-n-octylesterhydrogenmaleinat
Schmp. 89 - 91 °C.

Beispiel 48

2-[N-(1S-Methoxycarbonyl-3-phenylpropyl)-S-alanyl]-(1S,3S, 5S)-2-azabicyclo[3.3.0]octan-3-carbonsäure-n-octylester $[\alpha]_D^{25}$ , = -18,0° (c = 1, Methanol).

Beispiel 49

2-[N-(1S-Benzyloxycarbonyl-3-phenylpropyl)-S-alanyl]-(1S,3S, 5S)-2-azabicyclo[3.3.0]octan-3-carbonsäure-n-octylester $[\alpha]_D^{25}$ , = -34,3° (c = 1, Methanol).

Beispiel 50

2-[N-(1S-Carboxy-3-phenylpropyl)-S-alanyl]-(1S,3S,5S)-2-azabicyclo[3.3.0]octan-3-carbonsäure-n-decylester
Schmp. 52 °C.

**Ansprüche**

1. Verbindung der Formel I

$$R^3OOC-\overset{*}{\underset{R^4}{CH}}-\underset{R^5}{N}-\underset{O}{\overset{}{C}}-\overset{*}{\underset{R^1}{CH}}-NH-\overset{*}{\underset{COOR^2}{CH}}-(CH_2)_n-R \qquad (I)$$

in welcher
n = 2 ist
R einen gegebenenfalls substituierten aliphatischen Rest mit 1-18 C-Atomen,
einen gegebenenfalls substituierten alicyclischen Rest mit 3-20 C-Atomen
oder einen gegebenenfalls substituierten aromatischen Rest mit 6-12 C-Atomen bedeutet,
$R^1$ die erforderlichenfalls geschützte Seitenkette einer natürlich vorkommenden α-Aminosäure der Formel $R^1$-CH($NH_2$)-COOH bedeutet
$R^2$ Wasserstoff, einen gegebenenfalls substituierten aliphatischen Rest mit 1-18 C-Atomen, Benzyl oder

einen gegebenenfalls substituierten alicyclischen Rest mit 3-20 C-Atomen bedeutet,
$R^3$ Wasserstoff,
einen gegebenenfalls substituierten aliphatischen Rest mit 1-18 C-Atomen oder
einen gegebenenfalls substituierten araliphatischen Rest mit 7-20 C-Atomen bedeutet und
$R^4$ und $R^5$ zusammen mit den sie tragenden Atomen ein mono-, bi- oder tricyclisches heterocyclisches Ringsystem mit 3 bis 15 C-Atomen bilden,
vorausgesetzt daß
A
$R^2$ einen gegebenenfalls substituierten aliphatischen Rest mit 7-18 C-Atomen oder einen gegebenenfalls substituierten alicyclischen Rest mit 7-20 C-Atomen bedeutet und ansonsten n, R, $R^1$, $R^3$, $R^4$ und $R^5$ die oben angegebene Bedeutung besitzen,
oder
B
$R^3$ einen gegebenenfalls substituierten aliphatischen Rest mit 7-18 C-Atomen oder einen gegebenenfalls substituierten araliphatischen Rest mit 7-20 C-Atomen bedeutet und ansonsten n, R, $R^1$, $R^2$, $R^4$ und $R^5$ die oben angegebene Bedeutung besitzen,
oder
C
R einen gegebenenfalls substituierten aliphatischen Rest mit 9-18 C-Atomen bedeutet und ansonsten n, $R^1$, $R^2$, $R^3$, $R^4$ und $R^5$ die oben genannte Bedeutung besitzen,
oder deren physiologisch verträgliches Salz, ausgenommen
2-[N-(1S-Ethoxycarbonyl-3-phenylpropyl)-S-alanyl]-(1S,3S,5S)-2-azabicyclo[3.3.0]octan-3-carbonsäure-n-octylester,
2-[N-(1S-Carboxy-3-phenylpropyl)-S-alanyl]-(1S,3S,5S)-2-azabicyclo[3.3.0]octan-3-carbonsäure-n-octylester
und deren physiologisch verträgliche Salze.

2. Verbindung der Formel I gemäß Anspruch 1, in welcher
n = 2 ist,
R einen aliphatischen Rest mit 4-10 C-Atomen,
einen alicyclischen Rest mit 6 C-Atomen oder
einen aromatischen Rest mit 6 C-Atomen bedeutet,
$R^1$ Methyl oder Benzyl bedeutet,
$R^2$ Wasserstoff,
einen aliphatischen Rest mit 1-18 C-Atomen, Benzyl oder
einen alicyclischen Rest mit 6-10 C-Atomen bedeutet,
$R^3$ Wasserstoff,
einen aliphatischen Rest mit 2-14 C-Atomen oder
einen araliphatischen Rest mit 7-15 C-Atomen bedeutet und
$R^4$ und $R^5$ zusammen mit den sie tragenden Atomen ein mono-, bi- oder tricyclisches heterocyclisches Ringsystem mit 3-11 C-Atomen bilden
vorausgesetzt, daß
A
$R^2$ einen aliphatischen Rest mit 7-18 C-Atomen oder einen alicyclischen Rest mit 7-10 C-Atom n bedeutet und ansonsten n, R, $R^1$, $R^3$, $R^4$ und $R^5$ die oben angegebene Bedeutung besitzen,
oder
B
$R^3$ einen aliphatischen Rest mit 7-14 C-Atomen oder einen araliphatischen Rest mit 7-15 C-Atomen bedeutet und ansonsten n, R, $R^1$, $R^2$, $R^4$ und $R^5$ die oben angegebene Bedeutung besitzen,
oder
C
R einen aliphatischen Rest mit 9 oder 10 C-Atomen bedeutet und ansonsten n, $R^1$, $R^2$, $R^3$, $R^4$ und $R^5$ die oben angegebene Bedeutung besitzen,
oder deren physiologisch verträgliches Salz.

3. Verbindung der Formel I gemäß Anspruch 1 oder 2, in welcher
n = 2 ist,
R n-Butyl, n-Decyl, Cyclohexyl oder Phenyl bedeutet,
$R^1$ Methyl oder Benzyl bedeutet,
$R^2$ Wasserstoff, Methyl, Ethyl, Isobutyl, n-Octyl, n-Octadecyl, Benzyl oder Menthyl bedeutet,
$R^3$ Wasserstoff, Ethyl, n-Octyl, n-Nonyl, 5-Nonyl, n-Decyl, n-Tetradecyl, 2-Octenyl, 2-Octinyl, 3-Octinyl,

Benzyl, Benzhydryl oder 3,3-Diphenylpropyl bedeutet und
$R^4$ und $R^5$ zusammen mit den sie tragenden Atomen ein heterocyclisches Ringsystem aus der Reihe Octahydrocyclopenta[b]pyrrol, Spiro (bicyclo[2.2.2]octan)-2,3′-pyrrolidin], Pyrrolidin oder 1,2,3,3a,4,6a-Hexahydrocyclopenta[b] pyrrol bilden,
vorausgesetzt, daß
A
$R^2$ n-Octyl, n-Octadecyl oder Menthyl bedeutet und ansonsten n, R, $R^1$, $R^3$, $R^4$ und $R^5$ die oben angegebene Bedeutung besitzen, oder
B
$R^3$ n-Octyl, n-Nonyl, 5-Nonyl, n-Decyl, n-Tetradecyl, 2-Octenyl, 2-Octinyl, 3-Octinyl, Benzhydryl oder 3,3-Diphenylpropyl bedeutet und ansonsten n, R, $R^1$, $R^2$, $R^4$ und $R^5$ die oben angegebene Bedeutung besitzen oder
C
R n-Decyl bedeutet und ansonsten n, $R^1$, $R^2$, $R^3$, $R^4$ und $R^5$ die angegebene Bedeutung besitzen,
oder deren physiologisch verträgliches Salz.

4. Verbindung der Formel I gemäß einem oder mehrerer der Ansprüche 1 bis 3 aus der Gruppe der Verbindungen
2-[N-(1S-Ethoxycarbonyl-n-heptyl)-s-alanyl]-(1S,3S,5S)-2-azabicyclo[3.3.0]octan-3-carbonsäure-n-octylester;
2-[N-(1S-Ethoxycarbonyl-3-cyclohexylpropyl)-S-alanyl]-(1S,3S,5S)-2-azabicyclo[3.3.0]octan-3-carbonsäure-n-octylester;
2-[N-(1S-Ethoxycarbonyl-n-tridecyl)-S-alanyl]-(1S,3S,5S)-2-azabicyclo[3.3.0]octan-3-carbonsäure-benzylester;
2-[N-(1R-Ethoxycarbonyl-n-tridecyl)-S-alanyl]-(1S,3S,5S)-2-azabicyclo[3.3.0]octan-3 carbonsäure-benzylester;
2- N-(1S-Ethoxycarbonyl-3-phenylpropyl)-S-alanyl]-(1S,3S,5S)-2-azabicyclo[3.3.0]octan-3-carbonsäure-n-decylester;
2-[N-(1S-Ethoxycarbonyl-3-phenylpropyl)-S-alanyl]-(1S,3S,5S)-2-azabicyclo[3.3.0]octan-3-carbonsäure-(5-nonyl)-ester;
2-[N-(1S-Isobutyloxycarbonyl-3-phenylpropyl)-S-alanyl]-(1S,3S,5S)-2-azabicyclo[3.3.0]octan-3-carbonsäure-n-octylester;
2-[N-(1S-n-Octyloxycarbonyl-3-phenylpropyl)-S-alanyl]-(1S,3S,5S)-2-azabicyclo[3.3.0]octan-3-carbonsäure;
2-[N-(1S-n-Octyloxycarbonyl-3-phenylpropyl)-S-alanyl]-(1S,3S,5S)-2-azabicyclo[3.3.0]octan-3-carbonsäureethylester;
2-[N-(1S-n-Octyloxycarbonyl-3-phenylpropyl)-S-alanyl]-(1S,3S,5S)-2-azabicyclo[3.3.0]octan-3-carbonsäure-n-octylester;
2-[N-(1S-n-Octadecyloxycarbonyl-3-phenylpropyl)-S-alanyl]-(1S,3S,5S)-2-azabicyclo[3.3.0]octan-3-carbonsäurebenzylester;
2-[N-(1S-n-Octadecyloxycarbonyl-3-phenylpropyl)-S-alanyl]-(1S,3S,5S) 2-azabicyclo[3.3.0]octan-3-carbonsäuren-octylester;
2-[N-(1S-Menthyloxycarbonyl-3-phenylpropyl)-S-alanyl]-(1S,3S,5S)-2-azabicyclo[3.3.0]octan-3-carbonsäurebenzylester;
2-[N-(1S-Menthyloxycarbonyl-3-phenylpropyl)-S-alanyl]-(1S,3S,5S)-2-azabicyclo[3.3.0]octan-3-carbonsäure-benzhydrylester;
2-[N-(1S-Carboxy-3-phenylpropyl)-S-alanyl]-(1S,3S,5S)-2-azabicyclo[3.3.0]octan-3-carbonsäure-n-octylester;
2-[N-(1S-Ethoxycarbonyl-3-phenylpropyl)-S-alanyl]-(1S,3S,5S)-2-azabicyclo[3.3.0]-7-octen-3-carbonsäure-n-octylester;
N-[N-(1S-Ethoxycarbonyl-3-phenylpropyl)-S-alanyl]-S-prolin-n-octylester;
2-[N-(1S-Methoxycarbonyl-3-phenylpropyl)-S-alanyl]-(1S,3S, 5S)-2-azabicyclo[3.3.0]octan-3-carbonsäure-n-octylester;
2-|N-(1S-Benzyloxycarbonyl-3-phenylpropyl)-S-alanyl]-(1S,3S, 5S)-2-azabicyclo[3.3.0]octan-3-carbonsäure-n-octylester;
2-[N-(1S-Carboxy-3-phenylpropyl)-S-alanyl]-(1S,3S,5S)-2-azabicyclo[3.3.0]octan-3-carbonsäure-n-decylester
oder deren physiologisch verträgliches Salz.

5. Verfahren zur Herstellung einer Verbindung der Formel I gemäß einem der Ansprüche 1 bis 4, dadurch gekennzeichnet, daß man
a) eine Verbindung der Formel XIV

$$R-(CH_2)_n-\underset{COOQ^2}{\overset{*}{CH}}-NH-\underset{R^1}{\overset{*}{CH}}-\underset{O}{\overset{}{C}}-\underset{R^5}{N}-\underset{R^4}{\overset{*}{CH}}-COOQ^3 \qquad XIV)$$

umsetzt mit einer Verbindung der Formel XV

Q ≡ Y (XV)

wobei in den obengenannten Formeln XIV und XV R, $R^1$, $R^4$, $R^5$ und n die gleiche Bedeutung wie in Formel I haben und

i.

$Q^2$ wie $R^2$ in Formel I definiert ist, aber nicht Wasserstoff bedeutet, oder für eine basisch, sauer oder hydrogenolytisch leicht abspaltbare Carboxyl-Schutzgruppe steht,

$Q^3$ Wasserstoff bedeutet,

Y Hydroxy oder eine nucleophil verdrängbare Abgangsgruppe bedeutet und

Q wie $R^3$ in Formel I definiert ist, aber nicht Wasserstoff bedeutet, oder

Y $=\overset{(+)}{N}=\overset{(-)}{N}$ bedeutet und

Q einen Alkylidenrest mit 1 bis 18 C-Atomen bedeutet, oder

ii.

$Q^2$ Wasserstoff bedeutet,

$Q^3$ wie $R^3$ in Formel I definiert ist, aber nicht Wasserstoff bedeutet, oder für eine basisch, sauer oder hydrogenolytisch leicht abspaltbare Carboxyl-Schutzgruppe steht,

Y Hydroxy oder eine nucleophil verdrängbare Abgangsgruppe bedeutet und

Q wie $R^2$ in Formel I definiert ist, aber nicht Wasserstoff bedeutet, oder

Y $=\overset{(+)}{N}=\overset{(-)}{N}$ bedeutet und

Q einen Alkylidenrest mit 1 bis 18 C-Atomen bedeutet, oder daß man

b) eine Verbindung der Formel IX

$$R-(CH_2)_n-\underset{COOR^2}{\overset{*}{CH}}-OSO_2CF_3 \qquad (IX)$$

worin R, $R^2$ und n die gleiche Bedeutung wie in Formel I haben, mit einer Verbindung der Formel X

$$R^3OOC-\underset{R^4}{\overset{*}{CH}}-\underset{R^5}{N}-\underset{O}{C}-\underset{R^1}{\overset{*}{CH}}-NH_2 \qquad (X)$$

worin $R^1$, $R^3$, $R^4$ und $R^5$ die gleiche Bedeutung wie in Formel I haben, umsetzt oder daß man

c) eine Verbindung der Formel XI

$$R^3OOC-\underset{R^4}{\overset{*}{CH}}-\underset{R^5}{NH} \qquad (XI)$$

worin $R^3$, $R^4$ und $R^5$ die gleiche Bedeutung wie in Formel I haben, mit einer Verbindung der Formel XII

$$HOOC-\overset{*}{C}H(R^1)-NH-\overset{*}{C}H(COOR^2)-(CH_2)_n-R \qquad (XII)$$

worin n, R, $R^1$ und $R^2$ die gleiche Bedeutung wie in Formel I haben, in Analogie zu bekannten Peptidkupplungsverfahren umsetzt, in der so erhaltenen Verbindung, falls $Q^2$ oder $Q^3$ für eine basisch, sauer oder hydrogenolytisch abspaltbare Schutzgruppe steht, diese Schutzgruppe gegebenenfalls durch Behandeln mit einer Base, einer Säure oder durch Hydrieren abspaltet, und die auf obige Weise erhaltene Verbindung der Formel I gegebenenfalls in ihr physiologisch verträgliches Salz überführt.

6. Verbindung der Formel XI, in der $R^3$ n-Octyl, n-Nonyl, 5-Nonyl, n-Decyl, n-Tetradecyl, 2-Octenyl, 2-Octinyl, 3-Octinyl, Benzyl, Benzhydryl oder 3,3-Diphenylpropyl bedeutet und
$R^4$ und $R^5$ zusammen mit den sie tragenden Atomen ein heterocyclisches Ringsystem aus der Reihe Octahydrocyclopenta[b]pyrrol, Spiro[(bicyclo[2.2.2]octan)-2,3′-pyrrolidin], Pyrrolidin oder 1,2,3,3a,4,6a-Hexahydrocyclopenta[b]pyrrol bilden, sowie deren physiologisch verträgliche Salze, ausgenommen (1S,3S,5S)-2-Azabicyclo [3.3.0]octan-3-carbonsäure-n-octylester.

7. Verfahren zur Herstellung einer Verbindung der Formel XI gemäß Anspruch 6, dadurch gekennzeichnet, daß man eine Verbindung der Formel XI, worin $R^4$ und $R^5$ wie im Anspruch 6 definiert sind und $R^3$ Wasserstoff bedeutet, analog Anspruch 5, Variante a) umsetzt mit einer Verbindung der Formel XV, worin Q wie $R^3$ in Anspruch 6 definiert ist, aber nicht Wasserstoff bedeutet und Y Hydroxy oder eine nucleophil verdrängbare Abgangsgruppe bedeutet und die erhaltene Verbindung gegebenenfalls in ihr Salz überführt.

8. Verbindung gemäß einem der Ansprüche 1 bis 4 zur Anwendung als Heilmittel.

9. Verbindung gemäß einem der Ansprüche 1 bis 4 zur Anwendung als Heilmittel bei der Behandlung cognitiver Dysfunktionen.

10. Pharmazeutisches Mittel enthaltend eine wirksame Menge einer Verbindung gemäß einem der Ansprüche 1 bis 4 und einen physiologisch unbedenklichen Träger.

11. Verfahren zur Herstellung eines Mittels gemäß Anspruch 10, dadurch gekennzeichnet, daß man den Wirkstoff zusammen mit dem Träger und gegebenenfalls weiteren Zusatz-und/oder Hilfsstoffen in eine geeignete Darreichungsform bringt.

Patentansprüche für folgenden Vertragsstaat: ES

1. Verfahren zur Herstellung einer Verbindung der Formel I

$$R^3OOC-\overset{*}{C}H(R^4)-N(R^5)-C(=O)-\overset{*}{C}H(R^1)-NH-\overset{*}{C}H(COOR^2)-(CH_2)_n-R \qquad (I)$$

in welcher
n = 2 ist
R einen gegebenenfalls substituierten aliphatischen Rest mit 1-18 C-Atomen,
einen gegebenenfalls substituierten alicyclischen Rest mit 3-20 C-Atomen
oder einen gegebenenfalls substituierten aromatischen Rest mit 6-12 C-Atomen bedeutet,
$R^1$ die erforderlichenfalls geschützte Seitenkette einer natürlich vorkommenden α-Aminosäure der Formel $R^1$-$CH(NH_2)$-COOH bedeutet
$R^2$ Wasserstoff, einen gegebenenfalls substituierten aliphatischen Rest mit 1-18 C-Atomen, Benzyl oder einen gegebenenfalls substituierten alicyclischen Rest mit 3-20 C-Atomen bedeutet,
$R^3$ Wasserstoff,
einen gegebenenfalls substituierten aliphatischen Rest mit 1-18 C-Atomen oder
einen gegebenenfalls substituierten araliphatischen Rest mit 7-20 C-Atomen bedeutet und
$R^4$ und $R^5$ zusammen mit den sie tragenden Atomen ein mono-, bi- oder tricyclisches heterocyclisches Ringsystem mit 3 bis 15 C-Atomen bilden,
vorausgesetzt daß
A
$R^2$ einen gegebenenfalls substituierten aliphatischen Rest mit 7-18 C-Atomen oder einen gegebenenfalls substituierten alicyclischen Rest mit 7-20 C-Atomen bedeutet und ansonsten n, R, $R^1$, $R^3$, $R^4$ und $R^5$ die

oben angegebene Bedeutung besitzen,
oder
B
$R^3$ einen gegebenenfalls substituierten aliphatischen Rest mit 7-18 C-Atomen oder einen gegebenenfalls substituierten araliphatischen Rest mit 7-20 C-Atomen bedeutet und ansonsten n, R, $R^1$, $R^2$, $R^4$ und $R^5$ die oben angegebene Bedeutung besitzen,
oder
C
R einen gegebenenfalls substituierten aliphatischen Rest mit 9-18 C-Atomen bedeutet und ansonsten n, $R^1$, $R^2$, $R^3$, $R^4$ und $R^5$ die oben genannte Bedeutung besitzen,
oder deren physiologisch verträglichen Salzes, ausgenommen
2- N-(1S-Ethoxycarbonyl-3-phenylpropyl)-S-alanyl]-(1S,3S,5S)-2-azabicyclo[3.3.0]octan-3-carbonsäure-n-octylester;
2-[N-(1S-Carboxy-3-phenylpropyl)-S-alanyl]-(1S,3S,5S)-2-azabicyclo[3.3.0]octan-3-carbonsäure-n-octylester;
und dessen physiologisch verträglichen Salze, dadurch gekennzeichnet, daß man
a) eine Verbindung der Formel XIV

$$\begin{array}{llllllllllllll} & * & & & & * & & & & & & * & & \\ R-(CH_2)_n- & CH & - & NH & - & CH & - & C & - & N & - & CH & - & COOQ^3 \\ & | & & & & | & & \| & & | & & | & & \\ & COOQ^2 & & & & R^1 & & O & & R^5 & & R^4 & & \end{array} \qquad \text{XIV)}$$

umsetzt mit einer Verbindung der Formel XV
Q $=\!=\!=$ Y (XV)
wobei in den obengenannten Formeln XIV und XV R, $R^1$, $R^4$, $R^5$ und n die gleiche Bedeutung wie in Formel I haben und i.
$Q^2$ wie $R^2$ in Formel I definiert ist, aber nicht Wasserstoff bedeutet, oder für eine basisch, sauer oder hydrogenolytisch leicht abspaltbare Carboxyl-Schutzgruppe steht,
$Q^3$ Wasserstoff bedeutet,
Y Hydroxy oder eine nucleophil verdrängbare Abgangsgruppe bedeutet und
Q wie $R^3$ in Formel I definiert ist, aber nicht Wasserstoff bedeutet, oder
Y $=\overset{(+)}{N}$ $=\overset{(-)}{N}$ bedeutet und
Q einen Alkylidenrest mit 1 bis 18 C-Atomen bedeutet, oder
ii.
$Q^2$ Wasserstoff bedeutet,
$Q^3$ wie $R^3$ in Formel I definiert ist, aber nicht Wasserstoff bedeutet, oder für eine basisch, sauer oder hydrogenolytisch leicht abspaltbare Carboxyl-Schutzgruppe steht,
Y Hydroxy oder eine nucleophil verdrängbare Abgangsgruppe bedeutet und
Q wie $R^2$ in Formel I definiert ist, aber nicht Wasserstoff bedeutet, oder
Y $=\overset{(+)}{N}$ $=\overset{(-)}{N}$ bedeutet und
Q einen Alkylidenrest mit 1 bis 18 C-Atomen bedeutet, oder daß man
b) eine Verbindung der Formel IX

$$\begin{array}{llll} & * & & \\ R-(CH_2)_n- & CH & - & OSO_2CF_3 \\ & | & & \\ & COOR^2 & & \end{array} \qquad (IX)$$

worin R, $R^2$ und n die gleiche Bedeutung wie in Formel I haben, mit einer Verbindung der Formel X

$$\begin{array}{llllllllll} & * & & & & & & * & & \\ R^3OOC\ - & CH & - & N & - & C & - & CH & - & NH_2 \\ & | & & | & & \| & & | & & \\ & R^4 & & R^5 & & O & & R^1 & & \end{array} \qquad (X)$$

worin $R^1$, $R^3$, $R^4$ und $R^5$ die gleiche Bedeutung wie in Formel I haben, umsetzt
oder daß man

c) eine Verbindung der Formel XI

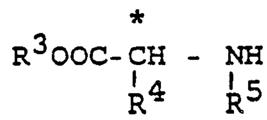

(XI)

worin $R^3$, $R^4$ und $R^5$ die gleiche Bedeutung wie in Formel I haben, mit einer Verbindung der Formel XII

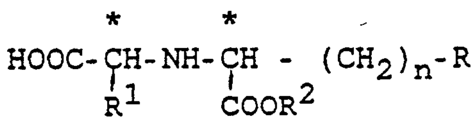

(XII)

worin n, R, $R^1$ und $R^2$ die gleiche Bedeutung wie in Formel I haben, in Analogie zu bekannten Peptidkupplungsverfahren umsetzt, in der so erhaltenen Verbindung, falls $Q^2$ oder $Q^3$ für eine basisch, sauer oder hydrogenolytisch abspaltbare Schutzgruppe steht, diese Schutzgruppe gegebenenfalls durch Behandeln mit einer Base, einer Säure oder durch Hydrieren abspaltet, und die auf obige Weise erhaltene Verbindung der Formel I gegebenenfalls in ihr physiologisch verträgliches Salz überführt.

2. Verfahren gemäß Anspruch 1, zur Herstellung einer Verbindung der Formel I, in welcher
n = 2 ist,
R einen aliphatischen Rest mit 4-10 C-Atomen,
einen alicyclischen Rest mit 6 C-Atomen oder
einen aromatischen Rest mit 6 C-Atomen bedeutet,
$R^1$ Methyl oder Benzyl bedeutet,
$R^2$ Wasserstoff,
einen aliphatischen Rest mit 1-18 C-Atomen, Benzyl oder
einen alicyclischen Rest mit 6-10 C-Atomen bedeutet,
$R^3$ Wasserstoff,
einen aliphatischen Rest mit 2-14 C-Atomen oder
einen araliphatischen Rest mit 7-15 C-Atomen bedeutet und
$R^4$ und $R^5$ zusammen mit den sie tragenden Atomen ein mono-, bi- oder tricyclisches heterocyclisches Ringsystem mit 3-11 C-Atomen bilden
vorausgesetzt, daß
A
$R^2$ einen aliphatischen Rest mit 7-18 C-Atomen oder einen alicyclischen Rest mit 7-10 C-Atomen bedeutet und ansonsten n, R, $R^1$, $R^3$, $R^4$ und $R^5$ die oben angegebene Bedeutung besitzen,
oder
B
$R^3$ einen aliphatischen Rest mit 7-14 C-Atomen oder einen araliphatischen Rest mit 7-15 C-Atomen bedeutet und ansonsten n, R, $R^1$, $R^2$, $R^4$ und $R^5$ die oben angegebene Bedeutung besitzen,
oder
C
R einen aliphatischen Rest mit 9 oder 10 C-Atomen bedeutet und ansonsten n, $R^1$, $R^2$, $R^3$, $R^4$ und $R^5$ die oben angegebene Bedeutung besitzen,
oder deren physiologisch verträglichen Salzes.

3. Verfahren gemäß Anspruch 1 oder 2, zur Herstellung von Verbindungen der Formel I, in welcher
n = 2 ist,
R n-Butyl, n-Decyl, Cyclohexyl oder Phenyl bedeutet,
$R^1$ Methyl oder Benzyl bedeutet,
$R^2$ Wasserstoff, Methyl, Ethyl, Isobutyl, n-Octyl, n-Octadecyl, Benzyl oder Menthyl bedeutet,
$R^3$ Wasserstoff, Ethyl, n-Octyl, n-Nonyl, 5-Nonyl, n-Decyl, n-Tetradecyl, 2-Octenyl, 2-Octinyl, 3-Octinyl, Benzyl, Benzhydryl oder 3,3-Diphenylpropyl bedeutet und
$R^4$ und $R^5$ zusammen mit den sie tragenden Atomen ein heterocyclisches Ringsystem aus der Reihe

Octahydrocyclopenta[b]pyrrol, Spiro[(bicyclo[2.2.2]octan)-2,3′-pyrrolidin], Pyrrolidin oder 1,2,3,3a,4,6a-Hexahydrocyclopenta[b] pyrrol bilden,
vorausgesetzt, daß
A
$R^2$ n-octyl, n-Octadecyl oder Menthyl bedeutet und ansonsten n, R, $R^1$, $R^3$, $R^4$ und $R^5$ die oben angegebene Bedeutung besitzen,
oder
B
$R^3$ n-octyl, n-Nonyl, 5-Nonyl, n-Decyl, n-Tetradecyl, 2-Octenyl, 2-Octinyl, 3-Octinyl, Benzhydryl oder 3,3-Diphenylpropyl bedeutet und ansonsten n, R, $R^1$, $R^2$, $R^4$ und $R^5$ die oben angegebenen Bedeutung besitzen
oder
C
R n-Decyl bedeutet und ansonsten n, $R^1$, $R^2$, $R^3$, $R^4$ und $R^5$ die angegebene Bedeutung besitzen,
oder deren physiologisch verträglichen Salzes.

4. Verfahren gemäß einem oder mehrerer der Ansprüche 1 bis 3 zur Herstellung einer Verbindungen aus der Gruppe
2-[N-(1S-Ethoxycarbonyl-n-heptyl)-S-alanyl]-(1S,3S,5S)-2-azabicyclo[3.3.0]octan-3-carbonsäure-n-octylester;
2-[N-(1S-Ethoxycarbonyl-3-cyclohexylpropyl)-S-alanyl]-(1S,3S,5S)-2-azabicyclo[3.3.0]octan-3-carbonsäure-n-octylester;
2-[N-(1S-Ethoxycarbonyl-n-tridecyl)-S-alanyl]-(1S,3S,5S)-2-azabicyclo[3.3.0]octan-3-carbonsäure-benzylester;
2-[N-(1R-Ethoxycarbonyl-n-tridecyl)-S-alanyl]-(1S,3S,5S)-2-azabicyclo[3.3.0]octan-3-carbonsäure-benzylester;
2-[N-(1S-Ethoxycarbonyl-3-phenylpropyl)-S-alanyl]-(1S,3S,5S)-2-azabicyclo[3.3.0]octan-3-carbonsäure-n-decylester;
2-[N-(Is-Ethoxycarbonyl-3-phenylpropyl)-S-alanyl]-(1S,3S,5S)-2-azabicyclo[3.3.0]octan-3-carbonsäure-(5-nonyl)-ester;
2-[N-(1S-Isobutyloxycarbonyl-3-phenylpropyl)-S-alanyl]-(1S,3S,5S)-2-azabicyclo[3.3.0]octan-3-carbonsäure-n-octylester;
2- N-(1S-n-Octyloxycarbonyl-3-phenylpropyl)-S-alanyl]-(1S,3S,5S)-2-azabicyclo[3.3.0]octan-3-carbonsäure;
2-[N-(1S-n-Octyloxycarbonyl-3-phenylpropyl)-S-alanyl]-(1S,3S,5S)-2-azabicyclo[3.3.0]octan-3-carbonsäureethylester;
2-[N-(1S-n-Octyloxycarbonyl-3-phenylpropyl)-S-alanyl]-(1S,3S,5S)-2-azabicyclo[3.3.0]octan-3-carbonsäure-n-octylester;
2-[N-(1S-n-Octadecyloxycarbonyl-3-phenylpropyl)-S-alanyl]-(1S,3S,5S)-2-azabicyclo[3.3.0]octan-3-carbonsäurebenzylester;
2-[N-(1S-n-Octadecyloxycarbonyl-3-phenylpropyl)-S-alanyl]-(1S,3S,5S)-2-azabicyclo[3.3.0]octan-3-carbonsäure-n-octylester;
2-[N-(1S-Menthyloxycarbonyl-3-phenylpropyl)-S-alanyl]-(1S,3S,5S)-2-azabicyclo[3.3.0]octan-3-carbonsäurebenzylester;
2-[N-(1S-Menthyloxycarbonyl-3-phenylpropyl)-S-alanyl]-(1S,3S,5S)-2-azabicyclo[3.3.0]octan-3-carbonsäurebenzhydrylester;
2-[N-(1S-carboxy-3-phenylpropyl)-S-alanyl]-(1S,3S,5S)-2-azabicyclo[3.3.0]octan-3-carbonsäure-n-octylester;
2-[N-(1S-Ethoxycarbonyl-3-phenylpropyl)-S-alanyl]-(1S,3S,5S)-2-azabicyclo[3.3.0]-7-octen-3-carbonsäure-n-octylester;
N-[N-(1S-Ethoxycarbonyl-3-phenylpropyl)-S-alanyl]-S-prolin-n-octylester;
2-[N-(1S-methoxycarbonyl-3-phenylpropyl)-S-alanyl]-(1S,3S, 5S)-2-azabicyclo[3.3.0]octan-3-carbonsäure-n-octylester;
2-[N-(1S-Benzyloxycarbonyl-3-phenylpropyl)-S-alanyl]-(1S,3S, 5S)-2-azabicyclo[3.3.0]octan-3-carbonsäure-n-octylester;
2-[N-(1S-Carboxy-3-phenylpropyl)-S-alanyl]-(1S,3S,5S)-2-azabicyclo[3.3.0]octan-3-carbonsäure-n-decylester.
oder deren physiologisch verträglichen Salzes.

5. Verfahren zur Herstellung einer Verbindung der Formel XI, in der
$R^{3'}$ n-Octyl, n-Nonyl, 5-Nonyl, n-Decyl, n-Tetradecyl, 2-Octenyl, 2-Octinyl, 3-Octinyl, Benzyl, Benzhydryl oder 3,3-Diphenylpropyl bedeutet und
$R^4$ und $R^5$ zusammen mit den sie tragenden Atomen ein heterocyclisches Ringsystem aus der Reihe Octahydrocyclopenta[b]pyrrol, Spiro (bicyclo[2.2.2]octan)-2,3′-pyrrolidin], Pyrrolidin oder 1,2,3,3a,4,6a-Hexahydrocyclopenta[b] pyrrol bilden,

oder deren physiologisch verträglichen Salzes, ausgenommen
(1S,3S,5S)-2-Azabicyclo[3.3.0]octan-3-carbonsäure-n-octylester,
dadurch gekennzeichnet, daß man eine Verbindung der Formel XI, worin $R^4$ und $R^5$ wie im Anspruch 5 definiert sind und $R^3$ Wasserstoff bedeutet, analog Anspruch 1, Variante a) umsetzt mit einer Verbindung der Formel XV, worin Q wie $R^3$ definiert ist, aber nicht Wasserstoff bedeutet und Y für Hydroxy oder eine nucleophil verdrängbare Abgangsgruppe bedeutet, und die erhaltene Verbindung gegebenenfalls in ihr Salz überführt.

6. Verfahren zur Herstellung eines pharmazeutisches Mittel enthaltend eine wirksame Menge einer Verbindung gemäß einem der Ansprüche 1 bis 4 und einen physiologisch unbedenklichen Träger, dadurch gekennzeichnet, daß man den Wirkstoff zusammen mit dem Träger und gegebenenfalls weiteren Zusatz- und/oder Hilfsstoffen in eine geeignete Darreichungsform bringt.

Patentansprüche für folgenden Vertragsstaat: GR

1. Verfahren zur Herstellung einer Verbindung der Formel I

$$R^3OOC - \underset{R^4}{\overset{*}{CH}} - \underset{R^5}{N} - \underset{O}{\overset{\|}{C}} - \underset{R^1}{\overset{*}{CH}} - NH - \underset{COOR^2}{\overset{*}{CH}} - (CH_2)_n - R \qquad (I)$$

in welcher
n = 2 ist
R einen gegebenenfalls substituierten aliphatischen Rest mit 1-18 C-Atomen,
einen gegebenenfalls substituierten alicyclischen Rest mit 3-20 C-Atomen
oder einen gegebenenfalls substituierten aromatischen Rest mit 6-12 C-Atomen bedeutet,
$R^1$ die erforderlichenfalls geschützte Seitenkette einer natürlich vorkommenden α-Aminosäure der Formel $R^1$-$CH(NH_2)$-COOH bedeutet
$R^2$ Wasserstoff, einen gegebenenfalls substituierten aliphatischen Rest mit 1-18 C-Atomen, Benzyl oder einen gegebenenfalls substituierten alicyclischen Rest mit 3-20 C-Atomen bedeutet,
$R^3$ Wasserstoff,
einen gegebenenfalls substituierten aliphatischen Rest mit 1-18 C-Atomen oder
einen gegebenenfalls substituierten araliphatischen Rest mit 7-20 C-Atomen bedeutet und
$R^4$ und $R^5$ zusammen mit den sie tragenden Atomen ein mono-, bi- oder tricyclisches heterocyclisches Ringsystem mit 3 bis 15 C-Atomen bilden,
vorausgesetzt daß
A
$R^2$ einen gegebenenfalls substituierten aliphatischen Rest mit 7-18 C-Atomen oder einen gegebenenfalls substituierten alicyclischen Rest mit 7-20 C-Atomen bedeutet und ansonsten n, R, $R^1$, $R^3$, $R^4$ und $R^5$ die oben angegebene Bedeutung besitzen,
oder
B
$R^3$ einen gegebenenfalls substituierten aliphatischen Rest mit 7-18 C-Atomen oder einen gegebenenfalls substituierten araliphatischen Rest mit 7-20 C-Atomen bedeutet und ansonsten n, R, $R^1$, $R^2$, $R^4$ und $R^5$ die oben angegebene Bedeutung besitzen,
oder
C
R einen gegebenenfalls substituierten aliphatischen Rest mit 9-18 C-Atomen bedeutet und ansonsten n, $R^1$, $R^2$, $R^3$, $R^4$ und $R^5$ die oben genannte Bedeutung besitzen,
oder deren physiologisch verträglichen Salzes, ausgenommen
2-[N-(1S-Ethoxycarbonyl-3-phenylpropyl)-S-alanyl]-(1S,3S,5S)-2-azabicyclo[3.3.0]octan-3-carbonsäure-n-octylester;
2-[N-(1S-Carboxy-3-phenylpropyl)-S-alanyl]-(1S,3S,5S)-2-azabicyclo[3.3.0]octan-3-carbonsäure-n-octylester;
und deren physiologisch verträglichen Salze, dadurch gekennzeichnet, daß man

a) eine Verbindung der Formel XIV

$$R-(CH_2)_n-\underset{\underset{COOQ^2}{|}}{\overset{*}{CH}}-NH-\underset{\underset{R^1}{|}}{\overset{*}{CH}}-\underset{\underset{O}{\|}}{C}-\underset{\underset{R^5}{|}}{N}-\underset{\underset{R^4}{|}}{\overset{*}{CH}}-COOQ^3 \qquad XIV)$$

umsetzt mit einer Verbindung der Formel XV

Q $=\!=\!=$ Y (XV)

wobei in den obengenannten Formeln XIV und XV R, $R^1$, $R^4$, $R^5$ und n die gleiche Bedeutung wie in Formel I haben und

i.

$Q^2$ wie $R^2$ in Formel I definiert ist, aber nicht Wasserstoff bedeutet, oder für eine basisch, sauer oder hydrogenolytisch leicht abspaltbare Carboxyl-Schutzgruppe steht,

$Q^3$ Wasserstoff bedeutet,

Y Hydroxy oder eine nucleophil verdrängbare Abgangsgruppe bedeutet und

Q wie $R^3$ in Formel I definiert ist, aber nicht Wasserstoff bedeutet, oder

Y $\overset{(+)}{=N}$ $\overset{(-)}{=N}$ bedeutet und

Q einen Alkylidenrest mit 1 bis 18 C-Atomen bedeutet, oder

ii.

$Q^2$ Wasserstoff bedeutet,

$Q^3$ wie $R^3$ in Formel I definiert ist, aber nicht Wasserstoff bedeutet, oder für eine basisch, sauer oder hydrogenolytisch leicht abspaltbare Carboxyl-Schutzgruppe steht,

Y Hydroxy oder eine nucleophil verdrängbare Abgangsgruppe bedeutet und

Q wie $R^2$ in Formel I definiert ist, aber nicht Wasserstoff bedeutet, oder

Y $\overset{(+)}{=N}$ $\overset{(-)}{=N}$ bedeutet und

Q einen Alkylidenrest mit 1 bis 18 C-Atomen bedeutet, oder daß man

b) eine Verbindung der Formel IX

$$R-(CH_2)_n-\underset{\underset{COOR^2}{|}}{\overset{*}{CH}}-OSO_2CF_3 \qquad (IX)$$

worin R, $R^2$ und n die gleiche Bedeutung wie in Formel I haben, mit einer Verbindung der Formel X

$$R^3OOC-\underset{\underset{R^4}{|}}{\overset{*}{CH}}-\underset{\underset{R^5}{|}}{N}-\underset{\underset{O}{\|}}{C}-\underset{\underset{R^1}{|}}{\overset{*}{CH}}-NH_2 \qquad (X)$$

worin $R^1$, $R^3$, $R^4$ und $R^5$ die gleiche Bedeutung wie in Formel I haben, umsetzt oder daß man

c) eine Verbindung der Formel XI

$$R^3OOC-\underset{\underset{R^4}{|}}{\overset{*}{CH}}-\underset{\underset{R^5}{|}}{NH} \qquad (XI)$$

worin $R^3$, $R^4$ und $R^5$ die gleiche Bedeutung wie in Formel I haben, mit einer Verbindung der Formel XII

$$\underset{R^1}{\overset{*}{HOOC-CH}}-NH-\underset{COOR^2}{\overset{*}{CH}}-(CH_2)_n-R \qquad (XII)$$

worin n, R, $R^1$ und $R^2$ die gleiche Bedeutung wie in Formel I haben, in Analogie zu bekannten Peptidkupplungsverfahren umsetzt, in der so erhaltenen Verbindung, falls $Q^2$ oder $Q^3$ für eine basisch, sauer oder hydrogenolytisch abspaltbare Schutzgruppe steht, diese Schutzgruppe gegebenenfalls durch Behandeln mit einer Base, einer Säure oder durch Hydrieren abspaltet, und die auf obige Weise erhaltene Verbindung der Formel I gegebenenfalls in ihr physiologisch verträgliches Salz überführt.

2. Verfahren gemäß Anspruch 1, zur Herstellung einer Verbindung der Formel I, in welcher
n = 2 ist,
R einen aliphatischen Rest mit 4-10 C-Atomen,
einen alicyclischen Rest mit 6 C-Atomen oder
einen aromatischen Rest mit 6 C-Atomen bedeutet,
$R^1$ Methyl oder Benzyl bedeutet,
$R^2$ Wasserstoff,
einen aliphatischen Rest mit 1-18 C-Atomen, Benzyl oder
einen alicyclischen Rest mit 6-10 C-Atomen bedeutet,
$R^3$ Wasserstoff,
einen aliphatischen Rest mit 2-14 C-Atomen oder
einen araliphatischen Rest mit 7-15 C-Atomen bedeutet und
$R^4$ und $R^5$ zusammen mit den sie tragenden Atomen ein mono-, bi- oder tricyclisches heterocyclisches Ringsystem mit 3-11 C-Atomen bilden
vorausgesetzt, daß
A
$R^2$ einen aliphatischen Rest mit 7-18 C-Atomen oder einen alicyclischen Rest mit 7-10 C-Atomen bedeutet und ansonsten n, R, $R^1$, $R^3$, $R^4$ und $R^5$ die oben angegebene Bedeutung besitzen, oder
B
$R^3$ einen aliphatischen Rest mit 7-14 C-Atomen oder einen araliphatischen Rest mit 7-15 C-Atomen bedeutet und ansonsten n, R, $R^1$, $R^2$, $R^4$ und $R^5$ die oben angegebene Bedeutung besitzen, oder
C
R einen aliphatischen Rest mit 9 oder 10 C-Atomen bedeutet und ansonsten n, $R^1$, $R^2$, $R^3$, $R^4$ und $R^5$ die oben angegebene Bedeutung besitzen,
oder deren physiologisch verträglichen Salzes.

3. Verfahren gemäß Anspruch 1 oder 2, zur Herstellung von Verbindungen der Formel I, in welcher
n = 2 ist,
R n-Butyl, n-Decyl, Cyclohexyl oder Phenyl bedeutet,
$R^1$ Methyl oder Benzyl bedeutet,
$R^2$ Wasserstoff, Methyl, Ethyl, Isobutyl, n-Octyl, n-Octadecyl, Benzyl oder Menthyl bedeutet,
$R^3$ Wasserstoff, Ethyl, n-Octyl, n-Nonyl, 5-Nonyl, n-Decyl, n-Tetradecyl, 2-Octenyl, 2-Octinyl, 3-Octinyl, Benzyl, Benzhydryl oder 3,3-Diphenylpropyl bedeutet und
$R^4$ und $R^5$ zusammen mit den sie tragenden Atomen ein heterocyclisches Ringsystem aus der Reihe Octahydrocyclopenta[b]pyrrol, Spiro[(bicyclo[2.2.2]octan)-2,3'-pyrrolidin], Pyrrolidin oder 1,2,3,3a,4,6a-Hexahydrocyclopenta[b] pyrrol bilden,
vorausgesetzt, daß
A
$R^2$ n-Octyl, n-Octadecyl oder Menthyl bedeutet und ansonsten n, R, $R^1$, $R^3$, $R^4$ und $R^5$ die oben angegebene Bedeutung besitzen, oder
B
$R^3$ n-Octyl, n-Nonyl, 5-Nonyl, n-Decyl, n-Tetradecyl, 2-Octenyl, 2-Octinyl, 3-Octinyl, Benzhydryl oder 3,3-Diphenylpropyl bedeutet und ansonsten n, R, $R^1$, $R^2$, $R^4$ und $R^5$ die oben angegebene Bedeutung besitzen oder
C
R n-Decyl bedeutet und ansonsten n, $R^1$, $R^2$, $R^3$, $R^4$ und $R^5$ die angegebene Bedeutung besitzen,
oder deren physiologisch verträglichen Salzes.

4. Verfahren gemäß einem oder mehrerer der Ansprüche 1 bis 3 zur Hersstellung einer Verbindung aus der Gruppe
2-[N-(1S-Ethoxycarbonyl-n-heptyl)-S-alanyl]-(1S,3S,5S)-2-azabicyclo[3.3.0]octan-3-carbonsäure-n-octylester;
2-[N-(1S-Ethoxycarbonyl-3-cyclohexylpropyl)-S-alanyl]-(1S,3S,5S)-2-azabicyclo[3.3.0]octan-3-carbonsäure-n-octylester;
2-[N-(1S-Ethoxycarbonyl-n-tridecyl)-S-alanyl]-(1S,3S,5S)-2-azabicyclo[3.3.0]octan-3-carbonsäure-benzylester;
2-[N-(1R-Ethoxycarbonyl-n-tridecyl)-S-alanyl]-(1S,3S,5S)-2-azabicyclo[3.3.0]octan-3-carbonsäure-benzylester;
2-[N-(1S-Ethoxycarbonyl-3-phenylpropyl)-S-alanyl]-(1S,3S,5S)-2-azabicyclo[3.3.0]octan-3-carbonsäure-n-decylester;
2-[N-(1S-Ethoxycarbonyl-3-phenylpropyl)-S-alanyl]-(1S,3S,5S)-2-azabicyclo[3.3.0]octan-3-carbonsäure-(5-nonyl)-ester;
2-[N-(1S-Isobutyloxycarbonyl-3-phenylpropyl)-S-alanyl]-(1S,3S,5S)-2-azabicyclo[3.3.0]octan-3-carbonsäure-n-octylester;
2-[N-(1S-n-Octyloxycarbonyl-3-phenylpropyl)-S-alanyl]-(1S,3S,5S)-2-azabicyclo[3.3.0]octan-3-carbonsäure;
2-[N-(1S-n-Octyloxycarbonyl-3-phenylpropyl)-S-alanyl]-(1S,3S,5S)-2-azabicyclo[3.3.0]octan-3-carbonsäureethylester;
2-[N-(1S-n-octyloxycarbonyl-3-phenylpropyl)-S-alanyl]-(1S,3S,5S)-2-azabicyclo[3.3.0]octan-3-carbonsäure-n-octylester;
2-[N-(1S-n-Octadecyloxycarbonyl-3-phenylpropyl-S-alanyl]-(1S,3S,5S)-2-azabicyclo[3.3.0]octan-3-carbonsäurebenzylester;
2-[N-(1S-n-Octadecyloxycarbonyl-3-phenylpropyl)-S-alanyl]-(1S,3S,5S)-2-azabicyclo[3.3.0]octan-3-carbonsäure-n-octylester;
2-[N-(1S-Menthyloxycarbonyl-3-phenylpropyl)-S-alanyl]-(1S,3S,5S)-2-azabicyclo[3.3.0]octan-3-carbonsäurebenzylester;
2-[N-(1S-Menthyloxycarbonyl-3-phenylpropyl)-S-alanyl]-(1S,3S,5S)-2-azabicyclo[3.3.0]octan-3-carbonsäurebenzhydrylester;
2-[N-(1S-Carboxy-3-phenylpropyl)-S-alanyl]-(1S,3S,5S)-2-azabicyclo[3.3.0]octan-3-carbonsäure-n-octylester;
2-[N-(1S-Ethoxycarbonyl-3-phenylpropyl)-S-alanyl]-(1S,3S,5S)-2-azabicyclo[3.3.0]-7-octen-3-carbonsäure-n-octylester;
N-[N-(1S-Ethoxycarbonyl-3-phenylpropyl)-S-alanyl]-S-prolin-n-octylester;
2-[N-(1S-Methoxycarbonyl-3-phenylpropyl)-S-alanyl]-(1S,3S, 5S)-2-azabicyclo[3.3.0]octan-3-carbonsäure-n-octylester;
2-[N-(1S-Benzyloxycarbonyl-3-phenylpropyl)-S-alanyl]-(1S,3S, 5S)-2-azabicyclo[3.3.0]octan-3-carbonsäure-n-octylester;
2-[N-(1S-carboxy-3-phenylpropyl)-S-alanyl]-(1S,3S,5S)-2-azabicyclo[3.3.0]octan-3-carbonsäure-n-decylester.
oder deren physiologisch verträgliches Salz.

5. Verbindung der Formel XI, in der
$R^3$ n-Octyl, n-Nonyl, 5-Nonyl, n-Decyl, n-Tetradecyl, 2-Octenyl, 2-Octinyl, 3-Octinyl, Benzyl, Benhydryl oder 3.3-Diphenylpropyl bedeutet und
$R^4$ und $R^5$ zusammen mit den sie tragenden Atomen ein heterocyclisches Ringsystem aus der Reihe Octahydrocyclopenta[b]pyrrol, Spiro[(bicyclo[2.2.2]octan)-2,3′-pyrrolidin], Pyrrolidin oder 1,2,3,3a,4,6a-Hexahydrocyclopenta[b] pyrrol bilden,
ausgenommen
(1S,3S,5S)-2-Azabicyclo[3.3.0]octan-3-carbonsäure-n-octylester.

6. Verfahren zur Herstellung einer Verbindung der Formel XI gemäß Anspruch 5, dadurch gekennzeichnet, daß man eine Verbindung der Formel XI, worin $R^4$ und $R^5$ wie oben definiert sind und $R^3$ Wasserstoff bedeutet, analog Anspruch 1, Variante a) umsetzt mit einer Verbindung der Formel XV, worin Q wie $R^3$ definiert ist, aber nicht Wasserstoff bedeutet und Y für Hydroxy oder eine nucleophil verdrängbare Abgangsgruppe bedeutet und die erhaltene Verbindung gegebenenfalls in ihr physiologisch verträgliches Salz überführt.

7. Verwendung einer Verbindung gemäß einem der Ansprüche 1 bis 4 als Heilmittel

8. Verwendung einer Verbindung gemäß einem der Ansprüche 1 bis 4 zur Anwendung als Heilmittel bei der Behandlung cognitiver Dysfunktionen.

9. Verfahren zur Herstellung eines pharmazeutisches Mittel enthaltend eine wirksame Menge iner Verbindung gemäß einem der Ansprüche 1 bis 4 und einen physiologisch unbedenklichen Träger, dadurch gekennzeichnet, daß man den Wirkstoff zusammen mit dem Träger und gegebenenfalls weiteren Zusatz- und/oder Hilfsstoffen in eine geeignete Darreichungsform bringt.